(19) Europäisches Patentamt
European Patent Office
Office européen des brevets

(11) Publication number: **0 319 325 A2**

(12) **EUROPEAN PATENT APPLICATION**

(21) Application number: **88311463.9**

(22) Date of filing: **02.12.88**

(51) Int. Cl.4: **G 03 F 7/10**

(30) Priority: **04.12.87 JP 306877/87**
**07.12.87 JP 309335/87**
**08.12.87 JP 310736/87**

(43) Date of publication of application:
**07.06.89 Bulletin 89/23**

(84) Designated Contracting States: **DE FR GB**

(71) Applicant: **WAKO PURE CHEMICAL INDUSTRIES, LTD.**
**10, Doshomachi-3-chome**
**Higashi-ku Osaka (JP)**

**Matsushita Electric Industrial Co., Ltd.**
**1006, Oaza Kadoma**
**Kadoma-shi Osaka-fu, 571 (JP)**

(72) Inventor: **Ogawa, Kazufumi**
**30-5 Higashinakaburi, 1-chome**
**Hirakata-shi (JP)**

**Ohno, Keiji**
**15-501, Higashisakado, 1-chome**
**Sakado-shi (JP)**

**Endo, Masayuki**
**782, Kurodoricho**
**Izumi-shi (JP)**

**Nagoya, Mamoru Kosumotopia 201**
**700-7, Tsurugaoka Tsurugashimamachi**
**Iruma-gun Saitama-ken (JP)**

(74) Representative: **Baillie, Iain Cameron et al**
**c/o Ladas & Parry Isartorplatz 5**
**D-8000 München 2 (DE)**

(54) **Photosensitive material and process for forming pattern using the same.**

(57) A photosensitive composition comprising a resin such as an alkali-soluble resin and a photosensitive compound such as a novel 3-diazo-2,4-dione derivative, a novel Meldrum's acid derivative, or the like is effective for forming a fine pattern using deep UV light of less than about 300 nm.

FIG. 2

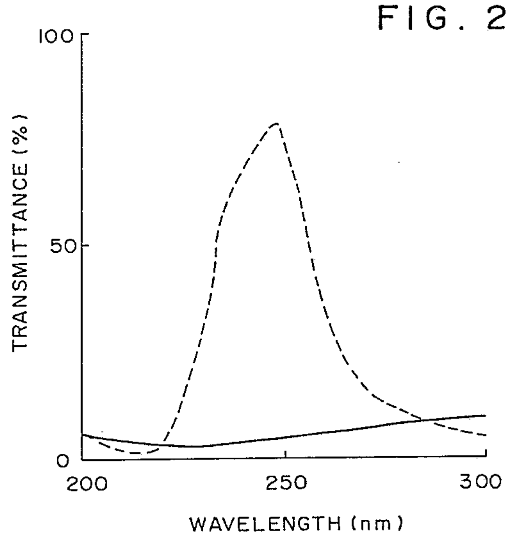


EP 0 319 325 A2

## Description

## PHOTOSENSITIVE MATERIAL AND PROCESS FOR FORMING PATTERN USING THE SAME

BACKGROUND OF THE INVENTION

This invention relates to a photosensitive material for lithography usable for mainly producing semiconductor devices and a process for forming a pattern using the same.

With recent higher density and larger scale integration of semiconductor devices, wavelengths used in exposing devices for minute processing, particularly for lithography become shorter and shorter. Now, KrF excimer laser light (248.4 nm) is studied. But, there have been no photosensitive materials suitable for such a wavelength.

For example, even when MP 2400 (mfd. by Shipley Corp.), which is known among photoresists to have high sensitivity to KrF excimer laser light and good light transmittance, is used, pattern formation after development is very bad and cannot be used practically [K. Ogawa, et al., J. Electrochem. Soc., 135, p. 2347 (1988)].

Pattern formation using MP 2400 and KrF excimer laser light is explained referring to Fig. 4. First, MP 2400 is spin coated on a Si substrate 1 and prebaked at 90°C for 2 minutes using a hot plate to obtain a resist film 3 of 1.2 µm thick [Fig. 4(a)]. Then, the resist film is selectively exposed to KrF excimer laser light 4 via a mask 5 [Fig. 4(b)]. Finally, development is conducted for 60 seconds using a 20% aqueous (alkaline) solution of MP 2401 (a trade name, mfd. by Shipley Corp.) to give a resin pattern 3a [Fig. 4(c)]. But when MP 2400 is used, since the sensitivity for the light of such a wavelength is bad, it is necessary to make the light exposing amount considerably large. As a result, the light enters into non-exposed portions to greatly deteriorate the shape of pattern 3a as shown in Fig. 4(c) and an aspect ratio becomes as low as about 60 degree. Such a bad shape of pattern seems to be derived from a large surface absorption of the MP 2400 resist film against the exposed light.

This can be interpreted that a main polymer (resin) per se used in the resist has a large light absorption against the exposed light, or a photosensitive material in the resist has no good light reactivity. That is, photosensitive materials such as naphthoquinone diazides heretofore used in known resists generally have a large absorption against a light near 248.4 nm and are hardly improved in transmittance after exposure to light. For example, in the case of MP 2400 with a film thickness of 1 µm, changes in light transmittance before and after exposure to light of KrF excimer laser (248.4 nm) is only about 3% at 248.4 nm as shown in Fig. 5 wherein the full line is before exposure and the dotted line is after exposure. This means that the transparency is poor.

On the other hand, U.S. Patent No. 4,622,283 to Gray discloses a lithographic resist composition for use with deep UV light of less than 300 nm wavelength containing as a photosensitive solubilizing agent a compound of the formula:

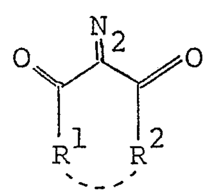

wherein $R^1$ and $R^2$ can each individually be alkyl, aryl, alkoxyalkyl, aralkyl or haloalkyl radicals or $R^1$ and $R^2$ taken together can be an alkylene radical. But this composition is insufficient in preventing non-light exposed portions from erosion of an alkaline developing solution to cause erosion of retaining portions of resist film (hereinafter referred to as "film erosion") in large amounts, which results in causing deterioration in contrast of the resist pattern.

Further, U.S. Patent No. 4,339,522 to Balanso et al disclose a lithographic resist composition comprising a phenolic-aldehyde resin and a deep ultraviolet sensitizer of the formula:

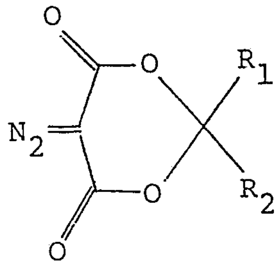

wherein $R_1$ is alkyl or aryl; and $R_2$ is H, alkyl or aryl, or together $R_1$ and $R_2$ are cycloalkyl. But this U.S. patent is quite silent on the reactivity against KrF excimer laser, resistance to alkaline developing solution, and improvement in the contrast.

SUMMARY OF THE INVENTION

It is an object of the present invention to provide a photosensitive composition having good reactivity against the deep ultraviolet light near 248.4 nm overcoming defects of known lithographic resist compositions.

It is another object of the present invention to provide a process for forming a fine pattern using such a photosensitive composition.

The present invention provides a photosensitive composition comprising a resin and a photosensitive compound of the formula:

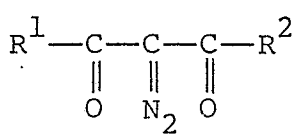

[I]

wherein $R^1$ is 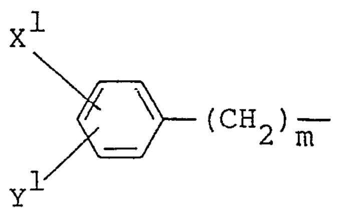 or 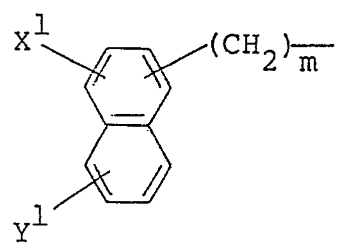 ;

$X^1$ and $Y^1$ are independently a hydrogen atom, a halogen atom, an alkyl group, an alkoxy group,

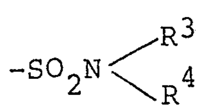 ,

$-SO_3R^5$ or a functional group reactive with the resin; $R^3$ and $R^4$ are independently a hydrogen atom or an alkyl group which may contain one or more functional groups, or $R^3$, $R^4$ and N taken together may form a heterocyclic ring; $R^5$ is an alkyl group; m is an integer of 1 or more; $R^2$ is an alkyl group, a cycloalkyl group, a hydroxyalkyl group an alkoxyalkyl group,

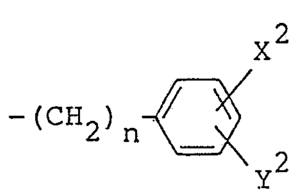 or

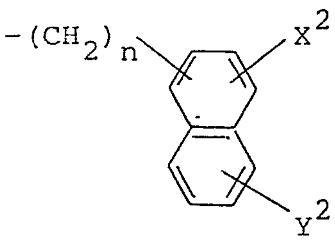 ;

$X^2$ and $Y^2$ are independently a hydrogen atom, a halogen atom, an alkyl group, an alkoxy group,

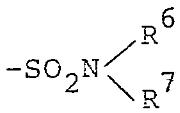 , $-SO_3R^8$

or a functional group reactive with the resin; $R^6$ and $R^7$ are independently a hydrogen atom or an alkyl group which may contain one or more functional groups, or $R^6$, $R^7$ and N taken together may form a heterocyclic ring; $R^8$ is an alkyl group; n is an integer of 1 or more; or $R^1$ and $R^2$ taken together may form a group of the formula:

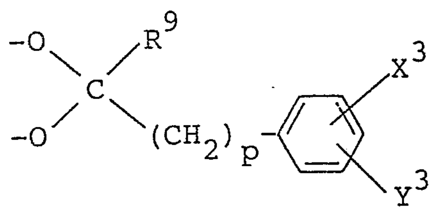

$R^9$ is an alkyl group, an aralkyl group, a hydroxyalkyl group or an alkoxyalkyl group; $X^3$ and $Y^3$ are independently a hydrogen atom, a halogen atom, an alkyl group, an alkoxy group,

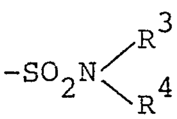 , $-SO_3R^5$,

or a functional group reactive with the resin; and p is an integer of 1 or more.

The present invention also provide a process for forming a fine pattern which comprises forming a film of a photosensitive composition comprising a resin and a photosensitive compound of the formula:

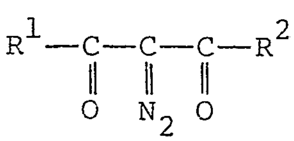 [I]

wherein $R^1$ and $R^2$ are as defined above, on a substrate,
exposing the film to deep UV light of less than about 300 nm, and
subjecting to development to form a pattern by removing exposed portions of the film.

## BRIEF DESCRIPTION OF THE DRAWINGS

Figs. 1(a) to (c) are cross-sectional views explaining one example of a pattern forming process according to the present invention.

Fig. 2 is a graph showing ultraviolet spectrophotometric characteristics of a photosensitive composition of the present invention at near 248.4 nm.

Figs. 3(a) to (d) are cross-sectional views explaining another example of a pattern forming process according to the present invention.

Figs. 4(a) to (c) are cross-sectional views explaining a pattern forming process according to a prior art process using MP 2400.

Fig. 5 is a graph showing ultraviolet spectrophotometric characteristics of MP 2400 at near 248.4 nm.

Figs. 6(a) to (c) are cross-sectional views explaining a further example of a pattern forming process according to the present invention.

Figs. 7 and 8 are graphs showing ultraviolet spectrophotometric characteristics of photosensitive compositions of the present invention.

## DESCRIPTION OF THE PREFERRED EMBODIMENTS

The photosensitive composition of the present invention comprises a resin and a photosensitive compound of the formula:

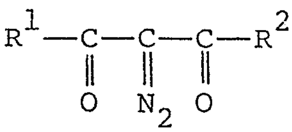

[I]

wherein $R^1$ is

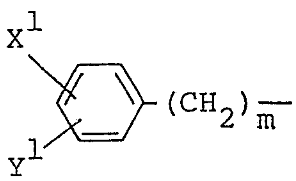

or

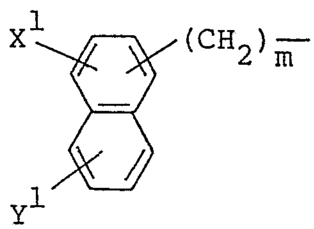

;

$X^1$ and $Y^1$ are independently a hydrogen atom, a halogen atom such as I, Cl, Br, or F, an alkyl group preferably having 1 to 5 carbon atoms, an alkoxy group preferably having 1 to 5 carbon atoms,

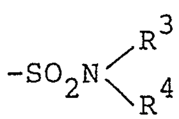

, $-SO_3R^5$

or a functional group reactive with the resin; $R^3$ and $R^4$ are independently a hydrogen atom or an alkyl group preferably having 1 to 5 carbon atoms and being able to have one or more functional groups, or $R^3$, $R^4$ and N taken together may form a heterocyclic ring such as a piperazine ring, a piperidine ring, a pyrrolidine ring, a morpholine ring, or the like; $R^5$ is an alkyl group preferably having 1 to 5 carbon atoms; m is an integer of 1 or more, preferably 1 to 20; $R^2$ is an alkyl group preferably having 1 to 20 carbon atoms, a cycloalkyl group preferably having 3 to 8 carbon atoms, a hydroxyalkyl group preferably having 1 to 20 carbon atoms, an alkoxyalkyl group preferably having 1 to 20 carbon atoms,

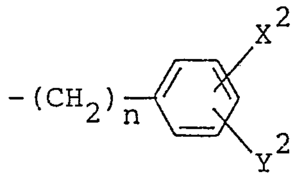

or

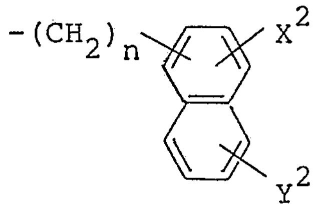

;

$X^2$ and $Y^2$ are independently a hydrogen atom, a halogen atom such as I, Cl, Br, or F, an alkyl group preferably having 1 to 5 carbon atoms, an alkoxy group preferably having 1 to 5 carbon atoms,

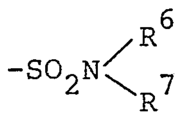

, $-SO_3R^8$

or a functional group reactive with the resin; $R^6$ and $R^7$ are independently a hydrogen atom or an alkyl group preferably having 1 to 5 carbon atoms and being able to have one or more functional groups, or $R^6$, $R^7$ and N taken together may form a heterocyclic ring such as a piperazine ring, a piperidine ring, a pyrrolidine ring, a morpholine ring, or the like; $R^8$ is an alkyl group preferably having 1 to 5 carbon atoms; m is an integer of 1 or more, preferably 1 to 20; n is an integer of 1 or more, preferably 1 to 20; or $R^1$ and $R^2$ taken together may form a group of the formula:

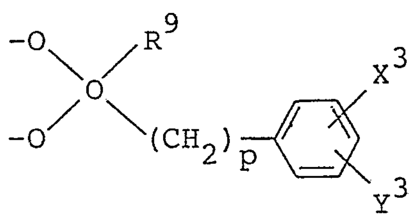

$R^9$ is an alkyl group preferably having 1 to 20 carbon atoms, an aralkyl group preferably having 7 to 20 carbon atoms such as a benzyl group, a phenethyl group, a phenylpropyl group, a phenylbutyl group, a phenylpentyl group, etc., a hydroxyalkyl group preferably having 1 to 20 carbon atoms, or an alkoxyalkyl group preferably having 1 to 20 carbon atoms; $X^3$ and $Y^3$ are independently a hydrogen atom, a halogen atom such as I, Cl, Br, or F, an alkyl group preferably having 1 to 5 carbon atoms, an alkoxy group preferably having 1 to 5 carbon atoms

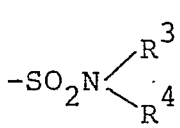

, $-SO_3R^5$ ,

or a functional group reactive with the resin; and p is an integer of 1 or more, preferably 1 to 20. The functional group reactive with the resin includes, for example, $-SO_2Cl$ (or a quaternary salt thereof), $-SO_3H$ (or an ammonium salt thereof, an organic base salt thereof, or a quaternary salt thereof), -COOH, -COCl, -COBr, $-NH_2$, or the like.

The compound of the formula [I] includes, for example, the following compounds.

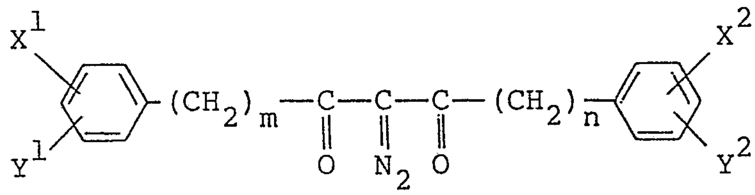

[II]

wherein $X^1$, $Y^1$, $X^2$, $Y^2$, m and n are as defined above,

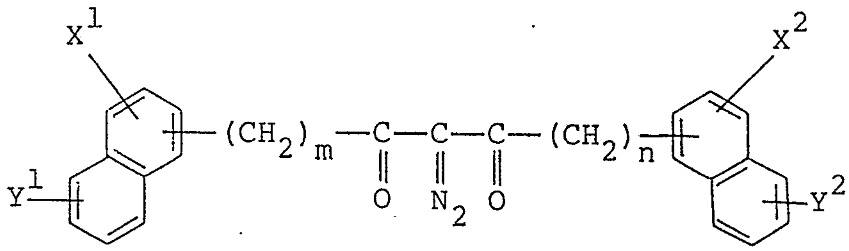

[III]

wherein $X^1$, $Y^1$, $X^2$, $Y^2$, m and n are as defined above,

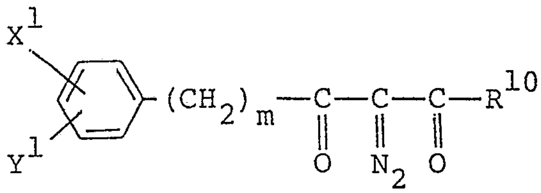

[IV]

wherein $R^{10}$ is an alkyl group preferably having 1 to 20 carbon atoms, a cycloalkyl group preferably having 3 to 8 carbon atoms, a hydroxyalkyl group preferably having 1 to 20 carbon atoms, or an alkoxyalkyl group preferably having 1 to 20 carbon atoms; $X^1$, $Y^1$ and m are as defined above, and

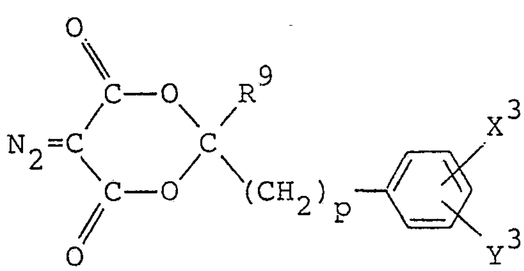

[V]

wherein $R^9$, $X^3$, $Y^3$ and p are as defined above.

The compound of the formula [II] can be formed, for example, as follows.

A compound of the formula:

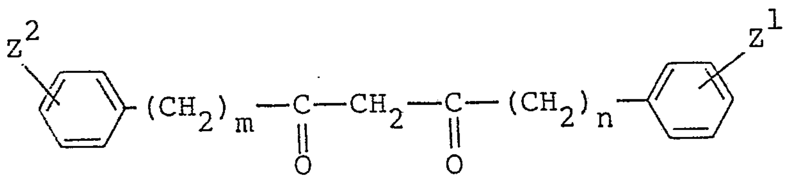 [VI]

wherein $Z^1$ and $Z^2$ are independently a hydrogen atome, a halogen atom such as I, Cl, Br, or F, an alkyl group preferably having 1 to 5 carbon atoms, or an alkoxy group preferably having 1 to 5 carbon atoms; m and n are as defined above, is reacted with a diazotizing agent such as tosyl azide in the presence of an organic base (e.g. pyridine, piperidine, triethylamine, morpholine, N-methylpyrrolidine, etc.) to give a compound of the formula [II] wherein $X^1$ and $X^2$ are hydrogen atoms; and $Y^1$, and $Y^2$ are independently a hydrogen atom, a halogen atom, an alkyl group, or an alkoxy group. This compound is reacted with chlorosulfonic acid to give a compound of the formula [II] wherein $X^1$ and $X^2$ are $-SO_2Cl$ or either one of $X^1$ and $X^2$ is $-SO_2Cl$, and another is a hydrogen atom; $Y^1$ and $Y^2$ are independently a hydrogen atom, a halogen atom, an alkyl group, or an alkoxy group (hereinafter defined as [II-2]). When this compound is hydrolyzed, there is obtained a compound of the formula [II] wherein $X^1$ and $X^2$ are $-SO_3H$ (or either one of $X^1$ and $X^2$ is $-SO_3H$ and the other is $-SO_2Cl$), or either one of $X^1$ and $X^2$ is $-SO_3H$ and another is a hydrogen atom; $Y^1$ and $Y^2$ are independently a hydrogen atom, a halogen atom, an alkyl group or an alkoxy group. When the compound of the formula [II-2] is subjected to alcoholysis using an alcohol such as methanol, ethanol, propanol, etc., in place of hydrolysis, there is obtained a compound of the formula [II] wherein $X^1$ and $X^2$ are $-SO_3R^5$ (or either one of $X^1$ and $X^2$ is $-SO_3R^5$ and the other is $-SO_2Cl$), or either one of $X^1$ and $X^2$ is $-SO_3R^5$ and another is a hydrogen atom; $Y^1$ and $Y^2$ are independently a hydrogen atom, a halogen atom, an alkyl group or an alkoxy group.

The compound of the formula [VI] can easily be produced by reacting a compound of the formula:

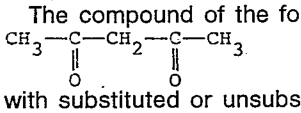 [VII]

with substituted or unsubstituted phenyl alkyl halides of the formula:

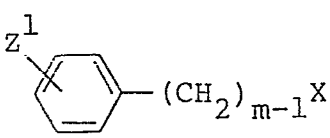 and 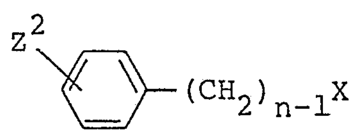

wherein $Z^1$, $Z^2$, m and n are as defined above; and X is Cl, Br or I, in the presence of a methallizing agent (e.g. n-butyllithium, lithium diisopropylamide, lithium 1,1,1,3,3,3-hexamethyldisilazane, etc.) at a temperature of 10°C or lower.

The compound of the formula [III] can be formed, for example, as follows.

A compound of the formula:

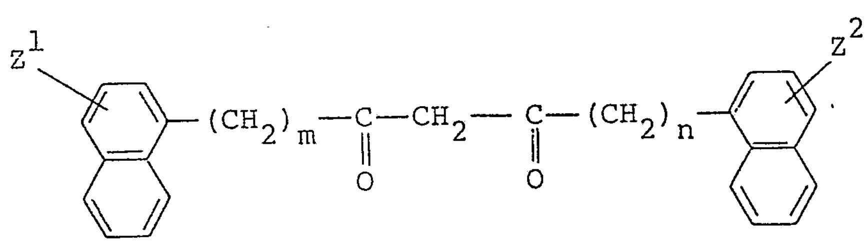 [VII]

wherein $Z^1$, $Z^2$, m and n are as defined above, is reacted with a diazotizing agent such as tosyl azide in the same manner as mentioned in the production of the compound of the formula [II].

The compound of the formula [IV] can be formed, for example, as follows.

A compound of the formula:

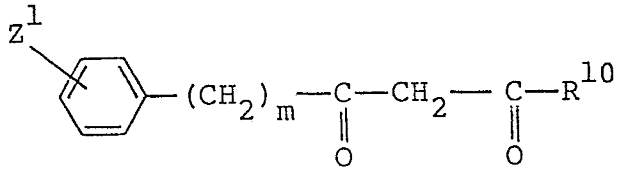

[VIII]

wherein $Z^1$, $R^{10}$ and m are as defined above, is reacted with a diazotizing agent such as tosyl azide in the presence of an organic base (e.g. pyridine, piperidine, triethylamine, morpholine, N-methylpyrrolidine, etc.) to give a compound of the formula [IV] wherein $X^1$ is a hydrogen atom and $Y^1$ is a hydrogen atom, a halogen atom, an alkyl group or an alkoxy group. This compound is reacted with chlorosulfonic acid to give a compound of the formula [IV] wherein $X^1$ is $-SO_2Cl$ and $Y^1$ is a hydrogen atom, a halogen atom, an alkyl group or an alkoxy group (herein after defined as [IV-2]). When the compound of the formula [IV-2] is hydrolyzed, there is obtained a compound of the formula [IV] wherein $X^1$ is $-SO_3H$ and $Y^1$ is a hydrogen atom, a halogen atom, an alkyl group or an alkoxy group. When the compound of the formula [IV-2] is subjected to alcoholysis using alcohol such as methanol, ethanol, propanol, etc., in place of hydrolysis, there is obtained a compound of the formula [IV] wherein $X^1$ is $-SO_3R^5$ and $Y^1$ is a hydrogen atom, a halogen atom, an alkyl group or an alkoxy group.

The compound of the formula [VIII] can easily be produced by either subjecting an ester of the formula:

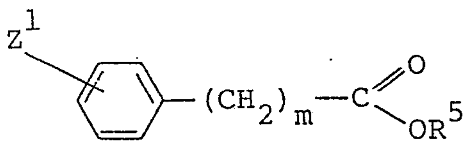

[IX]

wherein $Z^1$, $R^5$ and m are as defined above, and a ketone of the formula:

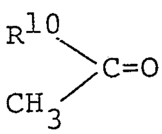

[X]

wherein $R^{10}$is as defined above, to a condensation reaction in the presence of metallic sodium, sodium hydride, or a metal alkoxide, or subjecting a ketone of the formula:

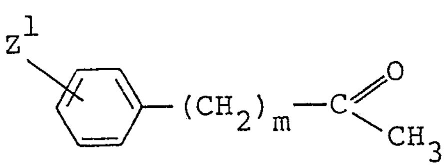

[XI]

wherein $Z^1$ and m are as defined above, and an ester of the formula:

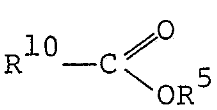

[XII]

wherein $R^5$ and $R^{10}$ are as defined above, to a condensaton reaction in the presence of methallic sodium, sodium hydride or a metal alkoxide.

The compound of the formula [V] can be formed, for example, as follows.

A compound of the formula:

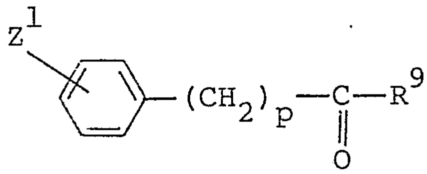

[XIII]

wherein $Z^1$, $R^9$ and p are as defined above, is reacted with malonic acid in the presence of acetic anhydride and sulfuric acid to give a compound of the formula:

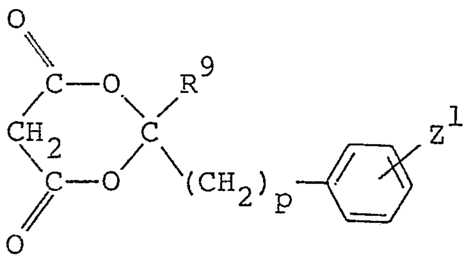

[XIV]

wherein $Z^1$, $R^9$ and p are as defined above. The compound of the formula [XIV] is reacted with a diazotizing agent such as tosyl azide in the presence of an organic base (e.g. pyridine, piperidine, triethylamine, morpholine, N-methylpyrrolidine, etc.) to give a Meldrum's acid derivative of the formula [V] wherein $X^3$ is a hydrogen atom and $Y^3$ is a hydrogen atom, a halogen atom, an alkyl group or an alkoxy group. This Meldrum's acid derivative is reacted with chlorosulfonic acid to give a Meldrum's acid derivative of the formula [V] wherein $X^3$ is $-SO_2Cl$ and $Y^3$ is a hydrogen atom ([V-2]). When the compound of the formula [V-2] is hydrolyzed, there is obtained a Meldrum's acid derivative of the formula [V] wherein $X^3$ is $-SO_3H$ and $Y^3$ is a hydrogen atom, a halogen atom, an alkyl group or an alkoxy group. When the compound of the formula [V-2] is subjected to alcoholysis using an alcohol such as methanol, ethanol, propanol, etc. in place of hydrolysis, there is obtained a Meldrum's acid derivative of the formula [V] wherein $X^3$ is $-SO_3R^5$; $Y^3$ is a hydrogen atom, a halogen atom, an alkyl group or an alkoxy group; and $R^5$ is as defined above.

As the resin which also consitutes the photosensitive composition, it is necessary to use resins having high transparency against deep UV light such as KrF excimer laser of 248.4 nm in order to reduce the surface absorption of light of the photosensitive composition. Examples of such resins are alkali-soluble resins such as p-cresol novolac resins, m-cresol novolac resins, maleimide resins, hydroxystyrene resins, o-chloro-m-cresol novolac resins, poly(p-vinylphenol), phenolic resins, styrene resins, maleic resins, copolymers of these monomers, etc.

The component ratio of the photosensitive compound to the resin in the resist (photosensitive composition) is 1:1 to 0.05:1 in weight ratio, preferably about 0.15:1. When the component ratio is about 0.15:1, photosensitivity of the resist is at the highest, and the highest aspect ratio resist pattern can be obtained.

The photosensitive compound of the formula [I] has a great reactivity against a light of 248.4 nm, that is, has a large change in light transmittance before and after exposure to the light (about 50% or more) and shows a transmittance of 70% or more after exposure to the light. Further, in a photosensitive composition comprising a compound of the formula [I] having a functional group reactive with a resin as at least one of $X^1$, $X^2$, $X^3$, $Y^1$, $Y^2$ and $Y^3$, and a resin having OH groups, when heated after coating of the photosensitive composition, a crosslinking reaction proceeds, which results in producing an effect for decreasing a solubility of the resin in a developing solution. Thus, by using such a photosensitive composition as a resist or the like material, there can be obtained a resin pattern having a good (non-eroded) shape.

That is, the group of the formula:

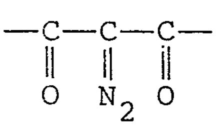

is changed to a group of the formula:

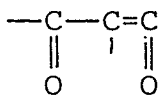

[XV]

by deep UV light such as KrF excimer laser light, or the like. By the action of an alkaline aqueous solution, the group of the formula [XV] changes as follows:

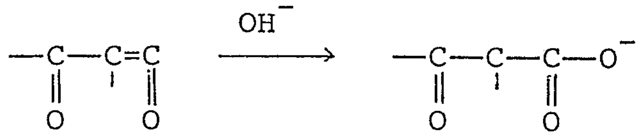

Thus, the photosensitive composition of the present invention becomes alkali-soluble in only light-exposed portions to form a so-called positive-type resist.

Generally speaking, a positive-type resist material is a combination of a photosensitive substance, a resin and a solvent. As the resin, an alkali-soluble novolac resin is maily used. Therefore, it is desirable that alkali solubility of non-light exposed portions of novolac resin is suppressed.

In the compound of the formula [I] having one or more functional groups reactive with the resin as at least one of $X^1$, $X^2$, $X^3$, $Y^1$, $Y^2$ and $Y^3$, since there are one or more functional groups such as $-SO_2Cl$, $-SO_2Br$, $-SO_3H$, -COOH, -COCl, -COBr, $-NH_2$, etc., at terminal groups, crosslinking proceeds with heating by forming, for example, ester bonds such as

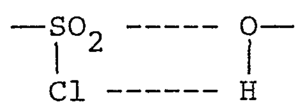

with -OH groups which are alkali-soluble portions of the resin, which results in suppressing alkali-solubility of the resin. Therefore, when the photosensitive composition of the present invention is used, the problem of erosion of retaining portions of resist film on the unexposed portions can be improved greatly.

Further, since the photosensitive compound of the formula [I] has one or two methylene chains such as $-(CH_2)_m-$ in the molecule, the molecule as a whole is stabilized, which results in improving thermal stability of the compound of the formula [I]. This also makes it possible to produce the compound of the formula [I] in industrially large amounts and to store the compound of the formula [I] stably for a long period of time.

In the compound of the formula [I], the position of functional group such as an alkyl, alkoxy, halogen

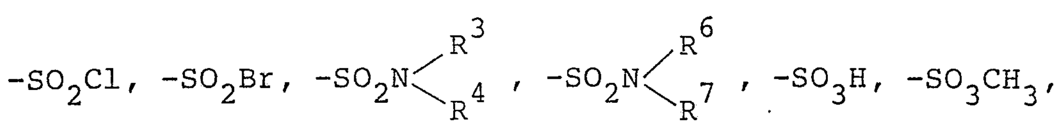

-COOH, -COCl, COBr, $-SO_3R^5$, $-SO_3R^8$,

or the like can be any one of o-, m- and p-position of a benzene ring with regard to the methylene group, or can be any one of 2- to 8-positions when the methylene group is attached to the 1-position of a naphthalene ring or any one of 1-position and 3- to 8-positions when the methylene group is attached to the 2-position of the naphthalene ring. In any cases, the effect as a photosensitizer is not influenced considerably. As to the length of the methylene group, the effect is not so influenced when m, n and p are in the range of 1 to 20. Generally speaking, when the methylene group chain becomes longer, there is a tendency to attain the same effect with a smaller amount and the solubility of the photosensitive compound of the formula [I] per se in a developing

solution is lowered. From this point of view, the longer the methylene group chain becomes, the better the results become.

The photosensitive composition can be used not only for producing semiconductor devices but also in various fields using light reactions such as production of printing materials and a photoengraving process as a photosensitive reagent.

The photosensitive composition is usually dissolved in an organic solvent to form a varnish. Examples of the organic solvent are those having high transparency such as diethylene glycol dimethyl ether, diethylene glycol diethyl ether, ethyl Cellosolve acetate, methyl Cellosolve acetate, etc.

When the photosensitive composition of the present invention is used for forming fine patterns, the following processes can be used.

One of the processes comprises forming a film of a photosenstive composition comprising a resin and a photosensitive compound of the formula [I] on a substrate, exposing the film to deep UV light of less than about 300 nm, and subjecting to development to form a pattern by removing exposed portions of the film. Concrete conditions are as follows:
Thickness of resist: 0.5-2 μm (0.1-0.5 μm in the case of the uppermost layer in a 3-layer structure)
Prebaking: 70° - 110°C for 10 - 30 minutes (in an oven) 70° - 110°C for 1 - 2 minutes (on a hot plate)
Exposure dose: 20 - 300 mJ/cm$^2$
Developing solution: an alkali solution (organic amines such as TMAH (tetramethylammonium hydroxide), inorganic alkalis e.g.. NaOH. KOH) in a concentration of usually 0.01 to 50%.
Developing time: 0.5 to 3 minutes (dipping, puddling, spraying, etc.)

Another process for forming a fine pattern is based on the use of the photosensitive composition as a contrast enhancing material for pattern formation. This process comprises forming a photoresist layer on a substrate, forming a contrast enhancing material layer comprising a resin and a photosensitive compound of the formula [I] on the photoresist layer, if necessary via a water-soluble organic film, exposing the contrast enhancing material layer to deep UV light of less than about 300 nm, removing the contrast enhancing material layer and the water-soluble organic film, and at the same time developing the photoresist layer to form a fine pattern. Concrete conditions are as follows:
Thickness of resist: 0.5-2 μm
Prebaking of resist: 70° - 110°C for 10 - 30 minutes (in an oven)
70° - 110°C for 1-2 minutes (on a hot plate)
Thickness of contrast enhancing material layer:
0.1 - 0.5 μm
Exposure dose: about 1.5 times as large as that of the lower resist layer.
Developing solution: an alkali solution (organic amines such as TMAH, inorganic alkalis e.g. NaOH, KOH) in a concentration of usually 0.01 to 50%
Developing time: 0.5 to 3 minues (dipping, puddling, spraying, etc.)

The present invention is illustrated by way of the following Examples, in which all percents are by weight unless oherwise specified.

Synthesis Example 1

(1) Synthesis of p-toluenesulfonyl azide (tosyl azide)

After dissolving 22.5 g (0.35 mole) of sodium axide in a small amount of water, the resulting solution was diluted with 30 ml of a 90% ethanol aqueous solution. To this, an ethanol solution dissolving 60 g (0.32 mole) of p-toluenesulfonyl chloride was added dropwise at 10 to 25°C, followed by reaction at room temperature for 2.5 hours. The reaction solution was concentrated at room temperature under reduced pressure. The resulting oily residue was washed with water several times and dried over anhydrous $MgSO_4$. After removing the drying agent by filtration, there was obtained 50.7 g of p-toluenesulfonyl azide as a colorless oil.
$^1$HNMR δppm ($CDCl_3$): 2.43 (3H, s, $\underline{CH_3}$), 7.24 (2H, d, J=8Hz, Ar 3-H, 5-H), 7.67 (2H, d, J=8Hz, Ar 2-H, 6-H).
IR (Neat) νcm$^{-1}$: 2,120 ($-N_3$).

(2) Synthesis of 1,7-diphenyl-3,5-heptadione

In 200 ml of cyclohexane, 17 g of 60% sodium hydride was placed and a cyclohexane solution dissolving 37 g (0.37 mole) of 2,4-pentanedione was added thereto dropwise at 20 to 25°C. The reaction was carried out at that temperature for 40 minutes. After changing 91.8 g of N,N,N′,N′-tetramethylethylenediamine, 337 g of n-hexane solution of n-butyllithium was added thereto dropwise at -5° to 0°C, followed by temperature rise to room temperature and 24 hours' reaction. Then, 93.7 g of benzyl chloride was added dropwise to the reaction solution at 0° to 5°C. After allowing to stand overnight, dilute hydrochloric acid was added thereto. After standing for separation of the reaction solution, an organic layer was separated, washed with water several times and dried over anhydrous $MgSO_4$. After removing the drying agent by filtration, the solvent was removed by distillation to give 79 g of a red oily material. After distillating under reduced pressure, 23.5 g of 1,7-diphenyl-3,5-heptadione having a boiling point of 185 to 190°C/0.4 mm Hg as a pale yellow oily material was obtained as a fraction.

(3) Synthesis of 4-diazo-1,7-diphenyl-3,5-heptanedione

In 120 ml of dichloromethane, 16.6 g (60 mmoles) of 1,7-diphenyl-3,5-heptadione obtained in above (2) was dissolved and 5.1 g (60 mmoles) of piperidine was added thereto. Then, 12.4 g (63 mmoles) of p-toluenesulfonyl azide obtained in above (1) was added dropwise to the resulting mixture at 0° to 5° C and the reaction was carried out at that temperature for 2 hours. Then, the reaction solution was washed with a dilute potassium hydroxide aqueous solution, washed with water several times, and dried over anhydrous $MgSO_4$. After removing the drying agent by filtration, the solvent was removed by distillation to give 19 g of a reddish orange oily material. Then, the resulting oily residue was subjected to column separation [silica gel, Wako Gel C-200, a trade name, mfd. by Wako Pure Chemical Industries, Ltd.; eluent, dichloromethane] to yield 8.0 g of 4-diazo-1,7-diphenyl-3,5-heptanedione as slightly yellow crystals having a melting point of 62.5 to 64.0°C.

$^1$HNMR δppm ($CDCl_3$): 2.97 (8H, m, -$\underline{CH_2}$-$X_4$), 7.20 (10H, s, ArH x 2)

IR (KBr-disk) $\nu cm^{-1}$: 2120 (-N2), 1650 ($\overline{C}$=0)

(4) Synthesis of 1,7-bis(4-chlorosulfonylphenyl)-4-diazo-3,5-heptanedione

To 22.4 g of chlorosulfonic acid, a chloroform solution dissolving 7.5 g (24 mmoles) of 4-diazo-1,7-diphenyl-3,5-heptanedione obtained in above (3) was added dropwise at 0° to 5°C. The reaction was carried out at that temperature for 1 hour. After pouring the reaction solution into 0.5 liter of ice water, the reaction solution was extracted with chloroform. The separated chloroform layer was washed with water several times and dried over anhydrous $MgSO_4$. After removing the drying agent by filtration, the solvent was removed by distillation to yield 5 g of an orange oily material as a residue. Then, the oily material was subjected to column separation [silica gel, Wako Gel C-200; eluent, n-hexane/methylene chloride=3/1 → 1/1(v/v)] to yield 3.0 g of 1,7-bis(4-chlorosulfonylphenyl)-4-diazo-3,5-heptanedione as yellow crystals having a melting point of 121° to 122.5°C.

$^1$HNMR δppm ($CDCl_3$):

3.10 (8H, s, -$\underline{CH_2}$- x 4), 7.40(4H,

d, J=8Hz, 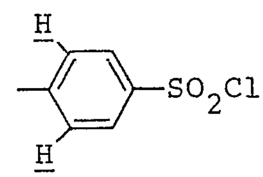 x 2),

7.53 (4H, d, J=8Hz, 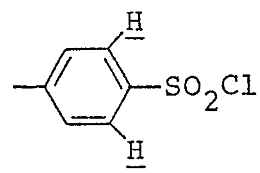

x 2)

IR (KBr-disk) $\nu cm^{-1}$: 2130 (-N2), 1640 (C=0)

Elementary analysis ($C_{19}H_{16}Cl_2N_2O_6S_2$):

| | C(%) | H(%) | N(%) |
|---|---|---|---|
| Calculated | 45.34 | 3.20 | 5.57 |
| Found | 45.25 | 3.36 | 5.56 |

Example 1

A photosensitive composition (1) was prepared by dissolving 3 g of 1,7-bis(4-chlorosulfonylphenyl)-4-diazo-3,5-heptanedione obtained in Synthesis Example 1(4) and 7 g of p-cresol novolac resin (molecular weight 10,000) in 50 g of diethylene glycol dimethyl ether with stirring.

Using the photosensitive composition (1), a pattern was formed as shown in Fig. 1. That is, the photosensitive composition (1) was spin coated on a substrate 1 such as a 6 inch silicon wafer in diameter and prebaked by a hot plate at 90°C for 2 minutes to give a photosensitive composition film 2 of 1.2 µm thick [Fig. 1(a)]. The film 2 was selectively exposed to KrF excimer laser light 4 of 248.4 nm via a mask 5 [Fig. 1(b)]. Then, development was carried out using a 2% alkaline aqueous solution of MP 2401 (a trade name, mfd. by Shipley Corp.) for 60 seconds to remove exposed portions of the film 2 by dissolution to give a resin pattern 2a [Fig. 1(c)]. The resin pattern had an aspect ratio of 90 degree with a good shape and was a submicron pattern with a film erosion of several percents or less. Using this pattern 2a as an etching mask, the surface of

substrate 1 was etched.

Ultraviolet spectrophotometric characteristics of the film 2 as a resist material were shown in Fig. 2, wherein the full line is before exposure and the dotted line is after exposure. The change of transmittance before and after exposure at 248.4 nm was as large as about 74%. This means that the photosensitive compound of the present invention shows good reactivity against the KrF excimer laser light.

Example 2

A photosensitive composition (2) was prepared bv dissolving 4 g of 1,7-bis(4-chlorosulfonylphenyl)-4-diazo-3,5-heptanedione obtained in Synthesis Example 1(4) and 8 g of m-cresol novolac resin (molecular weight 20,000) in 50 g of diethylene glycol dimethyl ether with stirring.

Using the photosensitive composition (2), a pattern was formed in the same manner as described in Example 1. The obtained pattern and the ultraviolet spectrophotometric characteristics were as good as those obtained in Example 1.

Example 3

A contrast enhancing material (1) for pattern formation was prepared by using the following ingredients:

| | |
|---|---|
| 1,7-Bis(4-chlorosulfonylphenyl)-4-diazo-3,5-heptanedione | 4.0 g |
| p-Cresol novolac resin (m.w. 5000) | 2.5 g |
| Ethyl Cellosolve acetate | 20.0 g |

A pattern forming organic film of 0.12 µm was formed using the contrast enhancing material (1). The difference in transmittance at 248.4 nm before and after exposure to KrF excimer laser light was as large as 50% or more. This shows that the contrast enhancing material (1) has a sufficient function for improving a contrast of incident light.

Using the contrast enhancing material (1), a resist pattern was formed by lithography as shown in Fig. 3.

A positive-type resist 3 (MP 2400, mfd. by Shipley Corp.) of 1.5 µm thick was spin coated on a substrate 1 such as a semiconductor [Fig. 3(a)]. Then, a water-soluble organic film 6 of 0.1 to 0.3 µm thick was formed on the positive-type resist 3 by coating a mixed solution of Plulan and polyvinyl pyrrolidone (4:1 in weight ratio). The water-soluble organic film is formed to separate the under layer of the resist 3 and an upper layer of pattern forming organic film 7 and is not always necessary [Fig. 3(b)]. Then, a layer 7 of the contrast enhancing material (1) for pattern formation of about 0.12 µm thick was formed by spin coating. The resist layer 3, the water-soluble organic film 6 and the contrast enhancing material layer 7 were laminated with good adhesion mutually. The layer 7 was selectively exposed to KrF excimer laser light 4 of 248.4 nm via a mask 5 using a projection aligner (5:1 reduction, NA=0.30) [Fig. 3(c)]. Using a conventional alkaline developing solution, the layer 7 and the water-soluble organic film 6 were removed simultaneously and at the same time the resist 3 was developed to form a resist pattern 3b [Fig. 3(d)]. The resist pattern 3b was improved in contrast (aspect ratio, 89 degree) and 0.3 µm lines and spaces were resolved.

Synthesis Example 2

(1) Synthesis of 1-phenyl-2,4-pentanedione

To 600 g (3.66 moles) of ethyl phenylacetate and 70.7 g (1.22 moles) of acetone, 28 g (1.22 atom%) of sodium was gradually added at 10° to 40°C. After stirring at 30° to 40°C for 1 hour for dissolution, the reaction was carried out at 70° to 80°C for 4.5 hours. After the reaction, 1.2 liters of water was added to the reaction solution, which was then neutralized by adding dilute hydrochloric acid thereto dropwise and extracted with chloroform. The separated chloroform layer was washed with water and dried over anhydrous $MgSO_4$. After removing the drying agent by filtration, the solvent was removed by distillation to yield 515 g of a reddish brown oily material. Then, the resulting residue was distilled under reduced pressure to yield 53.2 g of 1-phenyl-2,4-pentadione having a boiling point of 142 to 146°C/15 mm Hg as a colorless oily material.

(2) Synthesis of 3-diazo-1-phenyl-2,4-pentanedione

In 500 ml of methylene chloride, 36.2 g (0.21 mole) of 1-phenyl-2,4-pentanedione obtained in above (1) was dissolved, followed by addition of 17.5 g (0.21 mole) of piperidine. Then, 44.5 g (0.23 mole) of p-toluenesulfonyl azide obtained in Synthesis Example 1(1) was added thereto dropwise at 0° to 5°C, followed by reaction at that temperature for 1 hour. The reaction solution was washed with a dilute potassium hydroxide aqueous solution, washed with water and dried over anhydrous $MgSO_4$. After removing the drying agent by filtration, the solvent was removed by distillation to yield 50 g of a brown oily material. Then, the resulting oily residue was subjected to column separation [silica gel, Wako Gel C-200; eluent, n-hexane/ethyl acetate=10/1 (v/v)] to yield 33.0 g of 3-diazo-1-phenyl-2,4-pentanedione as an orange red viscous oily material.

$^1$HNMR δppm ($CDCl_3$): 2.36 (3H, s, $\underline{CH_3}$), 4.00 (2H, s, $\underline{-CH_2-}$), 7.23 (5H, s, aromatic ring)

IR (Neat): 2140 $cm^{-1}$ ($-N_2$), 1660 $cm^{-1}$ (C=0)

(3) Synthesis of 1-(4-chlorosulfonylphenyl)-3-diazo-2,4-pentanedione

In 50 ml of chloroform, 16.5 g (82 mmoles) of 3-diazo-1-phenyl-2,4-pentanedione obtained in above (2) was dissolved, followed by dropwise addition of 38 g of chlorosulfonic acid at -15° to -10°C. After stirring at -15° to -10°C for 2 hours, the reaction was further continued at 10° to 15°C for 3 hours. The reaction solution was poured into 1.5 liters of ice water, followed by extraction with chloroform. The separated chloroform layer was washed with water and dried over anhydrous $MgSO_4$. After removing the drying agent by filtration, the solvent was removed by distillation. The resulting reddish brown oily residue in an amount of 20 g was subjected to column separation [silica gel, Wako Gel C-200; eluent, n-hexane/ethyl acetate=5/1 (v/v)] to yield 10 g of reddish brown oily material. After recrystallizing from ethyl ether, there was obtained 4.0 g of 1-(4-chlorosulfonylphenyl)-3-diazo-2,4-pentanedione as yellow prism crystals having a melting point of 84.0 to 86.5°C (dec.).

$^1$HNMR δppm ($CDCl_3$): 2.45 (3H, s, $-\underline{CH_3}$), 4.30 (2H, s, $-\underline{CH_2}-$), 7.54 (2H, d, J=8Hz, phenyl-$C_2$, $C_6$), 8.00 (2H, d, J=8Hz, phenyl-$C_3$, $C_5$).

IR (KBr-disk): 2125 $cm^{-1}$ ($-N_2$), 1660 $cm^{-1}$ (C=0).

Elementary analysis ($C_{11}H_9ClN_2O_4S$)

| | C(%) | H(%) | N(%) |
|---|---|---|---|
| Calculated | 43.94 | 3.02 | 9.32 |
| Found | 44.02 | 3.14 | 9.30 |

Synthesis Example 3

(1) Synthesis of 6-phenyl-2,4-hexanedione

In 264 g of ethyl acetate, 148 g (1 mole) of benzyl acetone was dissolved, followed by gradual addition of sodium thereto at 20° to 60°C. After refluxing for further 2 hours with stirring, the reaction solution was cooled and poured into 1 liter of water. Then, the resulting solution was neutralized with concentrated hydrochloric acid by dropwise addition. After allowing to stand, the ethyl acetate layer was separated, washed with water and dried over anhydrous $MgSO_4$. After removing the drying agent, the solvent was removed by distillation to yield 171 g of a dark red oily material. The resulting oily residue was distilled under reduced pressure to give 73.4 g of 6-phenyl-2,4-hexanedione as a fraction having a boiling point of 128° to 133°C/3-4 mm Hg in coloreless oily state.

(2) Synthesis of 3-diazo-6-phenyl-2,4-hexanedione

In 300 ml of methylene chloride, 29.0 g (0.15 mole) of 6-phenyl-2,4-hexanedione obtained in above (1) was dissolved, followed by addition of 12.8 g (0.15 mole) of piperidine. Then, 31.2 g (0.16 mole) of p-toluenesulfonyl azide obtained in Synthesis Example 1(1) was added thereto dropwise at 0° to 5°C, followed by the reaction at that temperature for 1 hour. The reaction solution was washed with a dilute potassium hydroxide aqueous solution, washed with water and dried over anhydrous $MgSO_4$. After removing the drying agent by filtration, the solvent was removed by distillation to give 38 g of a dark red oily material. The resulting oily residue was subjected to column separation [silica gel, Wako Gel C-200; eluent, n-hexane/ethyl acetate=10/1 (v/v)] to yield 27.0 g of 3-diazo-6-phenyl-2,4-hexanedione as an ornge red viscous oily material. After cooling, there were obtained crystals having a melting point of 30.0° to 32.0°C.

$^1$HNMR δppm ($CDCl_3$): 2.47 (3H, s, $\underline{CH_3}$), 3.10 (4H, s, $-\underline{CH_2}-\underline{CH_2}-$), 7.28 (5H, s, aromatic ring).

IR (Neat): 2100 $cm^{-1}$ -N2), 1650 $cm^{-1}$ (C=0)

(3) Synthesis of 6-(4-chlorosulfonylphenyl)-3-diazo-2,4-hexanedione

In 35 ml of chloroform, 10.8 g (50 mmoles) of 3-diazo-6-phenyl-2,4-hexanedione obtained in above (2) was dissolved, followed by dropwise addition of 35 g of chlorosulfonic acid thereto at -10° to -5°C. After stirring at -5° to 0°C for 2 hours, the reaction solution was poured into 1 liter of ice water, followed by extraction with chloroform. The separated chloroform layer was washed with water and dried over anhydrous $MgSO_4$. After removing the drying agent by filtration, the solvent was removed by distillation. The resulting oily residue in an amount of 4 g was subjected to column separation [silica gel, Wako Gel C-200; eluent, n-hexane/ethyl acetate=5/1 (v/v)] to give 3.0 g of 6-(4-chlorosulfonylphenyl)-3-diazo-2,4-hexanedione as a yellow viscous oily material. After cooling, there were obtained crystals having a melting point of 58.0° to 59.5°C.

$^1$HNMR δppm ($CDCl_3$): 2.40 (3H, s, -$\underline{CH_3}$), 3.20 (4H, s, -$\underline{CH_2}$-$\underline{CH_2}$-CO-), 7.32 (2H, dd, J=8Hz, phenyl-$C_2$, $C_6$), 7.97 (2H, dd, J=8Hz, phenyl-$C_3$, $C_5$).
IR (Neat): 2130 $cm^{-1}$ (-$N_2$), 1660 $cm^{-1}$ (C=0)

Elementary analysis ($C_{12}H_{11}ClN_2O_4S$)

| | C(%) | H(%) | N(%) |
|---|---|---|---|
| Calculated | 45.65 | 3.51 | 8.87 |
| Found | 45.69 | 3.79 | 8.68 |

Example 4

A photosensitive composition (3) was prepared by dissolving 3 g of 1-(4-chlorosulfonylphenyl)-3-diazo-2,4-pentanedione obtained in Synthesis Example 2 (3) and 7 g of p-cresol novolac resin (molecular weight 10,000) in 50 g of diethylene glycol dimethyl ether with stirring.

Using the photosensitive composition (3), a pattern was formed in the same manner as described in Example 1. The resin pattern 2a had an aspect ratio of 87 degree with a good shape. The film erosion was several percents or less.

Ultraviolet spectrophotometric characteristics of the resulting film 2 (see Fig. 2) showed the change of transmittance before and after the exposure at 248.4 nm of as large as about 68%. This means that the photosensitive compound of the present invention shows good reactivity against the KrF excimer laser light.

Example 5

A photosensitive composition (4) was prepared by dissolving 4 g of 6-(4-chlorosulfonylphenyl)-3-diazo-2,4-hexanedione obtained in Synthesis Example 3 and 8 g of m-cresol novolac resin (molecular weight 20,000) in 42 g of ethyl Cellosolve acetate with stirring.

Using the photosensitive composition (4), a pattern was formed in the same manner as described in Example 1. The obtained pattern and the ultraviolet spectrophotometric characteristics were as good as those obtained in Example 4.

Example 6

A contrast enhancing material (2) for pattern formation was prepared by using the following ingredients:

| | |
|---|---|
| 6-(4-Chlorosulfonylphenyl)-3-diazo-2,4-hexanedione | 4.0 g |
| p-Cresol novolac resin (m.w. 5000) | 2.5 g |
| Ethyl Cellosolve acetate | 20.0 g |

A pattern forming organic film of 0.12 μm was formed using the contrast enhancing material (2). The difference in transmittance at 248.4 nm before and after exposure to KrF excimer laser light was as large as 50% or more. This shows that the contrast enhancing material (2) has a sufficient function for improving a contrast of incident light.

Using the contrast enhancing material (2), a resist pattern was formed by lithography in the same manner as described in Example 3.

The resulting resist pattern 3 b (see Fig. 3) was improved in contrast (aspect ratio, 89 degree) and 0.3 μm lines and spaces were resolved.

Synthesis Example 4

(1) Synthesis of 2-methyl-2-(2-phenylethyl)-1,3-dioxane-4,6-dione

To 56.7 g (0.55 mole) of malonic acid and 73 ml of acetic anhydride, 2 ml of concentrated sulfuric acid was added dropwise at 5° to 10°C. After stirring at that temperature for 20 minutes, 105 g (0.7 mole) of 4-phenyl-2-butanone was added thereto dropwise at 15° to 20°C, followed by stirring at that temperature for 15 hours. The reaction solution was gradually poured into water and extracted with chloroform. The separated chloroform layer was washed with water and dried over anhydrous $MgSO_4$. After removing the drying agent by filtration, the solvent was removed by distillation. The resulting pale brown crystals as a residue were recrystallized from water-containing acetone to yield 68.0 g of white powdery crystals of 2-methyl-2-(2-phenylethyl)-1,3-dioxane-4,6-dione having a melting point of 63.0° to 65.0°C.

$^1$HNMR δppm ($CDCl_3$): 1.74 (3H, s, $\underline{CH_3}$), 2.03-2.44 (2H, m, $-\underline{CH_2}-CH_2-$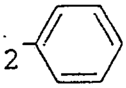), 2.60-3.02 (2H, m, $-CH_2-\underline{CH_2}-$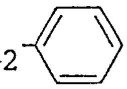), 3.58 (2H, s, 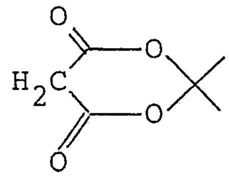), 7.16 (5H, s, aromatic ring)

IR (KBr-disk): 1750 $cm^{-1}$ (C=0)

(2) Synthesis of 5-diazo-2-methyl-2-(2-phenylethyl)-1,3-dioxane-4,6-dione

In 150 ml of ethanol, 67 g (0.29 mole) of 2-methyl-2-(2-phenylethyl)-1,3-dioxane-4,6-dione obtained in above (1) was dissolved, followed by dropwise addition of 32 g of triethylamine at -15° to -10°C. Then, 61 g (0.3 mole) of p-toluenesulfonyl azide obtained in Synthesis Example 1 (1) was added thereto dropwise at the same temperature. After stirring at that temperature for 45 minutes, the reaction solution was extracted with methylene chloride. The resulting organic layer was separated, washed with water and dried over anhydrous $MgSO_4$. After removing the drying agent by filtration, the solvent was removed by distillation. The resulting red oily residue was subjected to column separation [silica gel, Wako Gel C-200; eluent, n-hexane/ethyl acetate=10/1 . 5/1 - 3/1 (v/v)] to yield 44.0 g of 5-diazo-2-methyl-2-(2-phenylethyl)-1,3-dioxane-4,6-dione as yellow crystals having a melting point of 50.5° to 51.0°C.

$^1$HNMR δppm ($CDCl_3$): 1.74 (3H, s, $-\underline{CH_3}$), 2.04-2.47 (2H, m, $-\underline{CH_2}-CH_2-$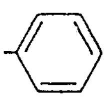), 2.60-3.02 (2H, m, ($-CH_2-\underline{CH_2}-$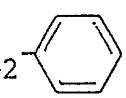), 7.16 (5H, s, aromatic ring).

IR (KBr-disk): 2180 $cm^{-1}$ ($-N_2$), 1695 $cm^{-1}$ (C=0)

(3) Synthesis of 2-[2-(4-chlorosulfonylphenyl)ethyl]-5-diazo-2-methyl-1,3-dioxane-4,6-dione

In 35 ml of chloroform, 8.0 g (31 mmoles) of 5-diazo-2-methyl-2-(2-phenylethyl)-1,3-dioxane-4,6-dione obtained in above (2) was dissolved, followed by dropwise addition into 21.5 g of chlorosulfonic acid at -20° to -10°C. After stirring at that temperature for 45 minutes, the reaction solution was poured into ice water, followed by extraction with chloroform. The resulting chloroform layer was separated, washed with water, and dried over anhydrous $MgSO_4$. After removing the drying agent by filtration, the solvent was removed by distillation. The resulting yellow oily residue in an amount of 5.3 g was subjected to column separation [silica gel, Wako Gel C-200; eluent, n-hexane/ethyl acetate=10/1 → 4/1 → 2/1 → 1/1 (v/v)] to yield 1.6 g of 2-[2-(4-chlorosulfonylphenyl)ethyl]-5-diazo-2-methyl-1,3-dioxane-4,6-dione as white powdery crystals having a melting point of 153.5° to 156.5°C.

$^1$HNMR δppm ($CDCl_3$): 1.82 (3H, s, $-\underline{CH_3}$), 2.13-2.56 (2H, m, $-\underline{CH_2}-CH_2-$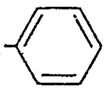), 2.73-3.19 (2H, m, $-CH_2-\underline{CH_2}-$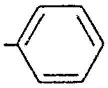), 7.44 (2H, d, J=9Hz, phenyl-$C_2$, $C_6$), 7.97 (2H, d, J=9Hz, phenyl-$C_3$, $C_5$).

IR (KBr-disk): 2170 $cm^{-1}$ ($-N_2$), 1710 $cm^{-1}$ (C=0)

Elementary analysis ($C_{13}H_{11}ClN_2O_6S$)

| | C(%) | H(%) | N(%) |
|---|---|---|---|
| Calculated | 43.52 | 3.09 | 7.81 |
| Found | 43.66 | 3.01 | 7.69 |

Example 7

A photosensitive composition (5) was prepared by dissolving 3 g of 2-[2-(4-chlorosulfonylphenyl)ethyl]-5-diazo-2-methyl-1,3-dioxane-4,6-dione obtained in Synthesis Example 4(3) and 7 g of p-cresol novolac resin (molecular weight 10,000) in 50 g of diethylene glycol dimethyl ether with stirring.

Using the photosensitive composition (5), a pattern was formed in the same manner as described in Example 1. The resin pattern 2a had an aspect ratio of 88 degree with a good shape. The film erosion was several percents or less.

Ultraviolet spectrophotometric characteristics of the resulting film 2 (see Fig. 2) showed the change of transmittance before and after the exposure at 248.4 nm of as large as about 71%. This means that the photosensitive compound of the present invention shows good reactivity against the KrF excimer laser light.

Example 8

A photosensitive composition (6) was prepared by dissolving 4 g of 2-[2-(4-chlorosulfonylphenyl)ethyl]-5-diazo-2-methyl-1,3-dioxane-4,6-dione obtained in Synthesis Example 4(3) and 8 g of m-cresol novolac resin (molecular weight 20,000) in 42 g of ethyl Cellosolve acetate with stirring.

Using the photosensitive composition (6), a pattern was formed in the same manner as described in Example 1. The obtained pattern and the ultraviolet spectrophotometric characteristics were as good as those obtained in Example 7.

Example 9

A contrast enhancing material (3) for pattern formation was prepared by using the following ingredients:

| | |
|---|---|
| 2-[2-(4-Chlorosulfonylphenyl)ethyl]-5-diazo-2-methyl-1,3-dioxane-4,6-dione | 4.0 g |
| p-Cresol novolac resin (m.w. 5000) | 2.5 g |
| Ethyl Cellosolve acetate | 20.0 g |

A pattern forming organic film of 0.12 µm was formed using the contrast enhancing material (3). The difference in transmittance at 248.4 nm before and after exposure to KrF excimer laser light was as large as 50% or more. This shows that the contrast enhancing material (3) has a sufficient function for improving a

contrast of incident light.

Using the contrast enhancing material (3), a resist pattern was formed by lithography in the same manner as described in Example 3.

The resulting resist pattern 3 b (see Fig. 3) was improved in contrast (aspect ratio, 89 degree) and 0.3 μm lines and spaces were resolved.

Example 10

A photosensitive composition (7) having the following composition was prepared:

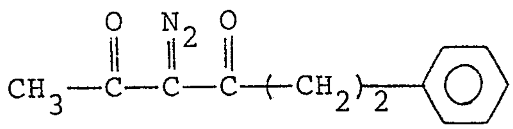 1.5 g

Poly(p-vinylphenol) (m.w. 7000) 8.5 g

Diethylene glycol dimethyl ether 30 g

The photosenstive composition (7) was spin coated on a semiconductor substrate (Si substrate) in 1.0 μm thickness and prebaked at 90° C using a hot plate for 2 minutes. Then, a dissolving rate in an alkali developing solution [a 2.38% aqueous solution of tetramethylammonium hydroxide (TMAH)] was measured. The dissolving rate was 400 nm/min, which value was 1/10 of that of poly(p-vinylphenol) (4000 nm/min). This means that the photosensitive compound mentioned above has a function of lowering the dissolving rate in the alkali developing solution.

A pattern was formed using the photosensitive composition (7) as shown in Fig. 6. A layer 2 of the photosensitive composition (7) was formed by spin coating on a substrate 1 such as a semiconductor in 1.0 μm thickness [Fig. 6(a)]. The substrate 1 may have thereon an oxidizing film, an insulating film, and an electroconductive film. The layer 2 was selectively exposed to KrF excimer laser 4 via a mask 5 [Fig. 6(b)]. Finally, the layer 2 was dipped in a 2.38% TMAH aqueous solution for 1 minute to form a pattern 2a by dissolving and removing the exposed portions [Fig. 6(c)]. The resulting pattern 2a had a film erosion of about 40%, an aspect ratio of 70 degree and resolution of 0.4 μm lines and spaces.

Example 11

A photosensitive composition (8) having the following composition was prepared:

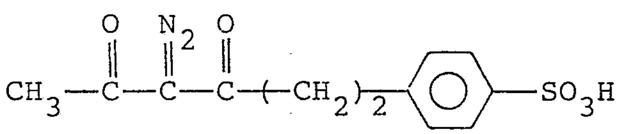 1.5 g

Poly(p-vinylphenol) 8.5 g

Diethylene glycol dimethyl ether 30 g

The photosensitive composition (8) was subjected to the same test and pattern formation as in Example 10. The dissolving rate was 200 nm/min, which value was better than that of Example 10. Further, the film erosion was 30%, the aspect ratio was 80 degree, and 0.4 μm lines and spaces were resolved.

Example 12

A photosensitive composition (9) having the following composition was prepared:

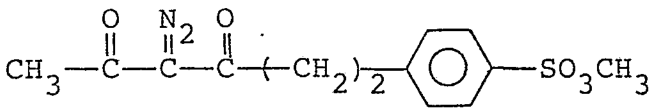

1.5 g

| | |
|---|---|
| Poly(p-vinylphenol) | 8.5 g |
| Diethylene glycol dimethyl ether | 30.0 g |

The photosensitive composition (9) was subjected to the same test and pattern formation as in Example 10. The dissolving rate was 200 nm/min which value was the same as Example 11. The formed pattern was as good as that of Example 11.

Example 13

A photosensitive composition (10) having the following composition was prepared:

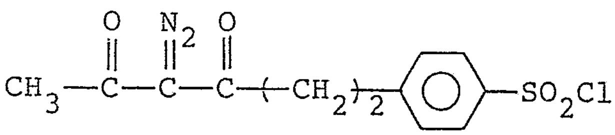

1.5 g

| | |
|---|---|
| Poly(p-vinylphenol) | 8.5 g |
| Diethylene glycol dimethyl ether | 30.0 g |

The photosensitive composition (10) was subjected to the same test and pattern formation as in Example 10. The dissolving rate was as good as 50 nm/min. Further, the film erosion was 5%, the aspect ratio was 82 degree, and 0.4 µm lines and spaces were resolved.

As shown in Examples 11 to 13, by adding $-SO_3H$, $-SO_3CH_3$, or $-SO_2Cl$ group to a terminal of a photosensitive compound, the film erosion at the pattern formation can be suppressed. Particularly, the photosensitive compound having a $-SO_2Cl$ group is possible to suppress the dissolution remarkably by bonding to the -OH group of the resin.

Example 14

A photosensitive composition (11) having the following composition was prepared:

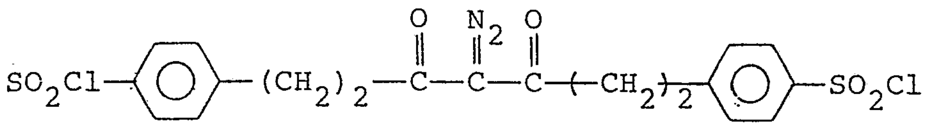 1.5 g

Poly(p-vinylphenol) 8.5 g

Diethylene glycol dimethyl ether 30.0 g

The photosensitive composition (11) was subjected to the same test and pattern formation as in Example 10. The dissolving rate was 10 nm/min or less, which value was very excellent. The film erosion was hardly observed, the aspect ratio was 86 degree or more, and 0.4 µm lines and spaces were resolved.

Ultraviolet spectrophotometric characteristics of a thin film of the photosensitive composition (11) formed on a silica substrate in 1.0 µm thickness before and after exposure to KrF excimer laser light of 248.4 nm were shown in Fig. 7, wherein the full line is before exposure and the dotted line is after exposure. As is clear from Fig. 7, good light fading properties are shown therein.

By adding two or more $SO_2Cl$ groups to one molecule, the ester bond mentioned above is generated at least 2 positions in the molecule, which results in further reducing the film erosion of non-exposed portions due to the formation of higher molecular weight of the resin via the photosensitive compound.

Example 15

A photosensitive composition (12) having the following composition was prepared:

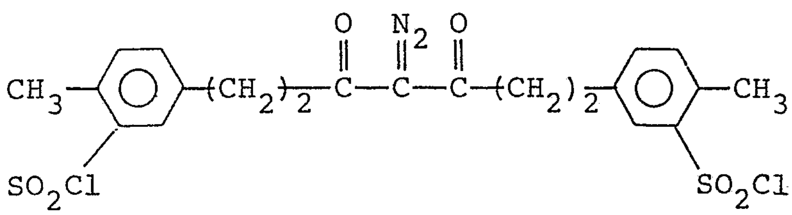 1.5 g

Poly(p-vinylphenol) 8.5 g

Diethylene glycol dimethyl ether 30.0 g

The photosensitive composition (12) was subjected to the same test and pattern formation as in Example 10. The dissolving rate was 10 nm/min or less, which value was very excellent. In the pattern formation, there was obtained a pattern without the film erosion, and with the aspect ratio of 90 degree, resolution of 0.4 µm lines and spaces and high sensitivity about twice as large as that of Example 14.

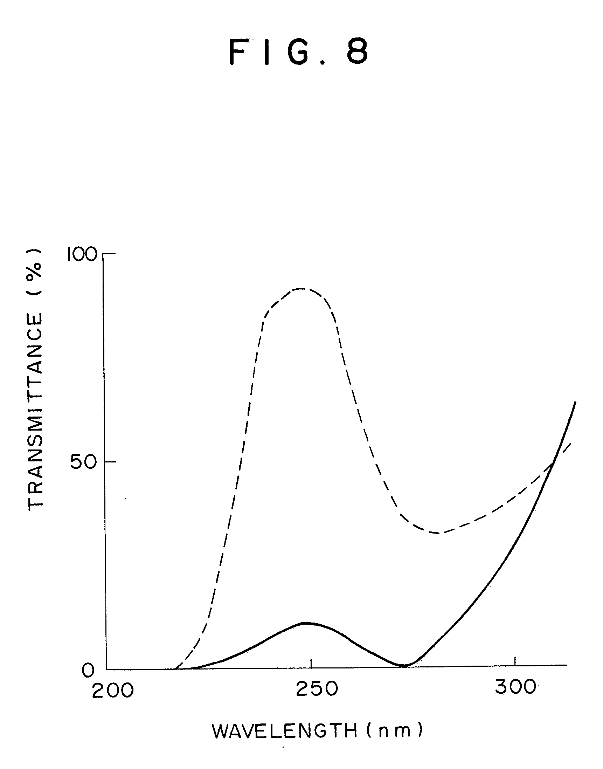

Ultraviolet spectrophotometric characteristics of a thin film of the photosensitive composition (12) formed on a silica substrate in 1.0 µm thickness before and after exposure to KrF excimer laser light of 248.4 nm were shown in Fig. 8, wherein the full line is before exposure and the dotted line is after exposure. Comparing with Fig. 7, the photosensitive composition (12) is improved in the transmittance at 248.4 nm both in before and after exposure compared with the photosensitive composition (11).

These results seem to show that by improving the transmittance before exposure, the light readily reaches the lower portion of the photosensitive composition to increase the sensitivity, and that by improving the transmittance after exposine, the pattern formation improved in the aspect ratio seems to become possible.

Example 16

A photosensitive composition (13) having the following composition was prepared:

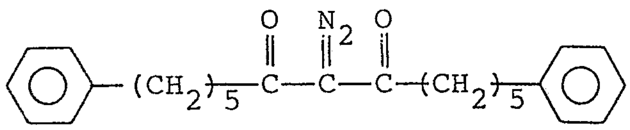

1.5 g

Poly(p-vinylphenol) 8.5 g

Diethylene glycol dimethyl ether 30.0 g

The photosensitive composition (13) was subjected to the same test and pattern formation as in Example 10. The dissolving rate was 100 nm/min, which value was 1/3 of that of Example 10. The film erosion was 10%, the aspect ratio was 80 degree, and 0.4 µm lines and spaces were resolved.

As shown above, by making the alkyl chain in the photosensitive compound long, the dissolution of the photosensitive compound per se in the developing solution is lowered. As a result, the film erosion is also lowered.

Example 17

A photosensitive composition (14) having the following composition was prepared:

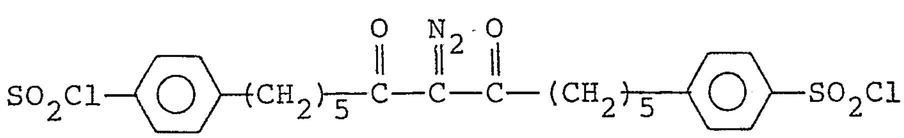

1.0 g

Poly(p-vinylphenol) 9.0 g

Diethylene glycol dimethyl ether 30.0 g

The photosensitive composition (14) was subjected to the same test and pattern formation as in Example 10. The dissolving rate was 10 nm/min or less, although the content of the photosensitive compound was smaller than that in Example 14. The film erosion was not observed, the aspect ratio was 90 degree, and 0.4 µm lines and spaces were resolved.

As a result of ultraviolet spectrophotometric analysis, the transmittances at 248.4 nm before and after exposure were higher than those of Example 14.

Example 18

A photosensitive composition (15) having the following composition was prepared:

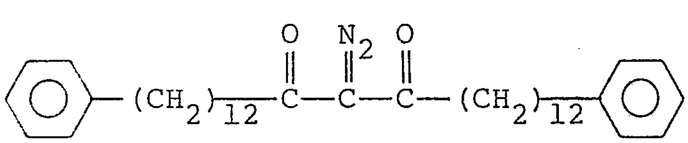

1.0 g

Poly(p-vinylphenol) 9.0 g

Diethylene glycol dimethyl ether 30.0 g

The photosensitive composition (15) was subjected to the same test and pattern formation as in Example 10. The dissolving rate was 10 nm/min or less, although the content of the photosensitive compound was smaller than that in Example 16. The film erosion was not observed, the aspect ratio was 90 degree, and 0.4 µm lines and spaces were resolved.

Example 19

A photosensitive composition (16) having the following composition was prepared:

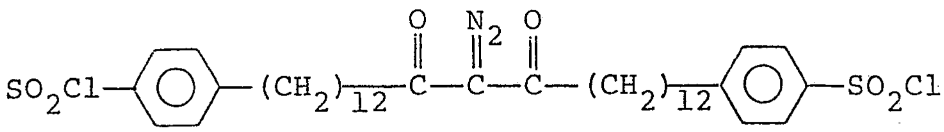 0.6 g

Poly(p-vinylphenol) 9.4 g

Diethylene glycol dimethyl ether 30.0 g

The photosensitive composition (16) was subjected to the same test and pattern formation as in Example 10. The dissolving rate was 10 nm/min or less, although the content of the photosensitive compound was smaller than that in Example 17. The film erosion was not observed, the aspect ratio was 90 degree, and 0.4 µm lines and spaces were resolved.

As a result of ultraviolet spectrophotometric analysis, the transmittances at 248.4 nm before and after exposure were higher than those of Example 17.

Example 20

A photosensitive composition (17) having the following composition was prepared:

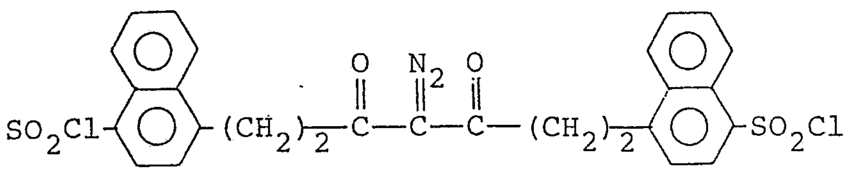 1.0 g

Poly(p-vinylphenol) 9.0 g

Diethylene glycol dimethyl ether 30.0 g

The photosensitive composition (17) was subjected to the same test and pattern formation as in Example 10. The dissolving rate was 10 nm/min or less, although the content of the photosensitive compound was smaller than that in Example 14. The film erosion was not observed, the aspect ratio was 90 degree, and 0.4 µm lines and spaces were resolved.

Example 21

A photosensitive composition (18) having the following composition was prepared:

| | |
|---|---|
| 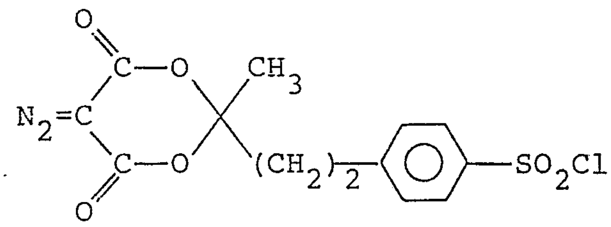 | 1.5 g |
| Poly(p-vinylphenol) | 8.5 g |
| Diethylene glycol dimethyl ether | 30.0 g |

The photosensitive composition (18) was subjected to the same test and pattern formation as in Example 10. The dissolving rate was 50 nm/min. The film erosion was 5%, the aspect ratio was 83 degree, and 0.4 μm lines and spaces were resolved.

As mentioned above, by applying the photosensitive compound and the photosensitive composition including the photosensitive compound to photoresist for deep UV light less than 300 nm, e.g. KrF excimer laser light (248.4 nm), and contrast enhancing materials, fine patterns of submicron order with good shape can easily be obtained.

Example 22

Photosensitive compositions comprising a photosensitive compound of the formula (I) as listed in Table 1, poly(p-vinylphenol) as a polymer and diethylene glycol dimethyl ether as a solvent, in amounts as listed in Table 1 were prepared.

Each photosensitive composition was subjected to the same dissolving test and pattern formation as in Example 10.

The dissolving rate, the film erosion, the aspect ratio and the resolution were measured in the same manner as in Example 10 and listed in Table 1.

Table 1

| Run No. | Compound (I) | Mixing ratio (parts) | | |
|---|---|---|---|---|
| | | Compound (I) | Polymer | Solvent |
| 1 | 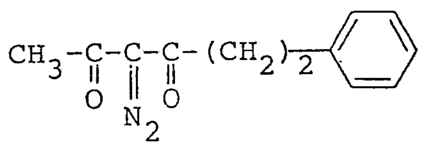 | 15 | 85 | 300 |
| 2 | 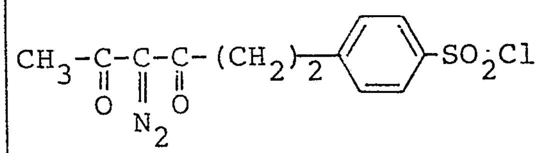 | " | " | " |
| 3 | 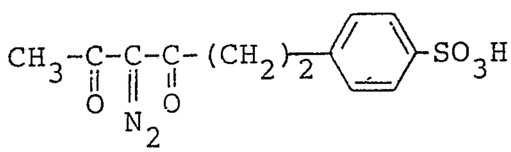 | " | " | " |
| 4 | 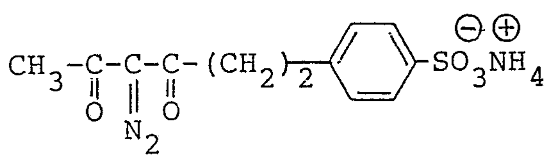 | " | " | " |
| 5 | 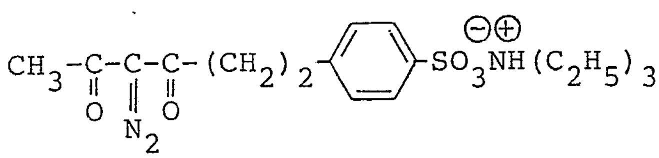 | " | " | " |
| 6 | 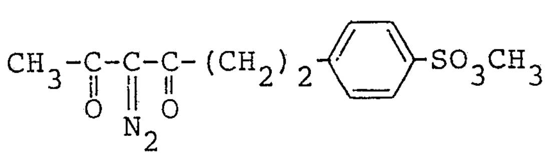 | " | " | " |

(To be cont'd)

Table 1 (Cont'd)

| Dissolving rate (nm/min) | Film erosion (%) | Aspect ratio (degree) | Resolution (lines and spaces) (μm) |
|---|---|---|---|
| 400 | 40 | 70 | 0.4 |
| 150 | 15 | 82 | 0.4 |
| 200 | 30 | 80 | 0.4 |
| 200 | 30 | 80 | 0.4 |
| 200 | 30 | 80 | 0.4 |
| 200 | 30 | 80 | 0.4 |

(To be cont'd)

Table 1 (Cont'd)

| | | | | |
|---|---|---|---|---|
| 7 | 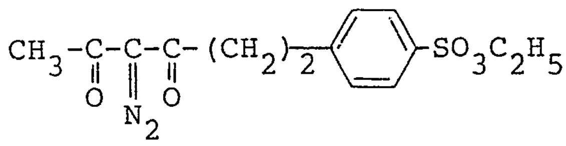 | 15 | 85 | 300 |
| 8 | 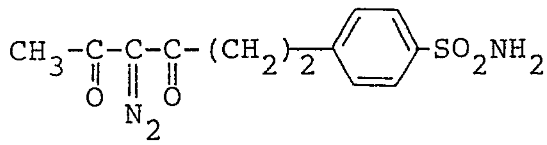 | " | " | " |
| 9 | 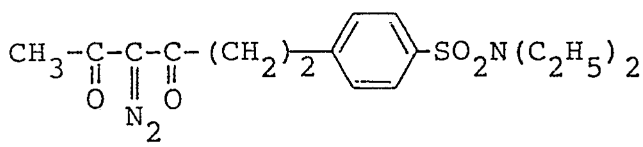 | " | " | " |
| 10 | 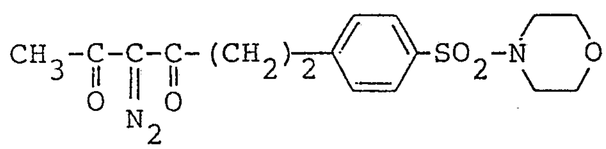 | " | " | " |
| 11 | 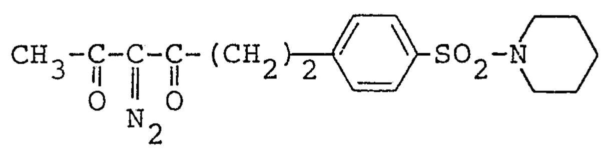 | " | " | " |
| 12 | 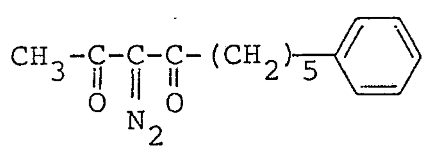 | " | " | " |
| 13 | 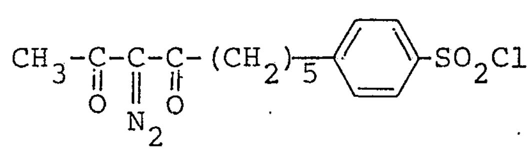 | " | " | " |

(To be cont'd)

Table 1 (Cont'd)

| | | | |
|---|---|---|---|
| 170 | 25 | 81 | 0.4 |
| 200 | 25 | 80 | 0.4 |
| 180 | 20 | 81 | 0.4 |
| 200 | 25 | 80 | 0.4 |
| 200 | 25 | 80 | 0.4 |
| 120 | 12 | 83 | 0.4 |
| 80 | 10 | 85 | 0.4 |

(To be cont'd)

Table 1 (Cont'd)

| | | | | |
|---|---|---|---|---|
| 14 | 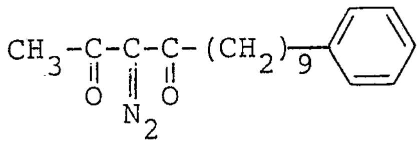 | 12 | 88 | 300 |
| 15 | 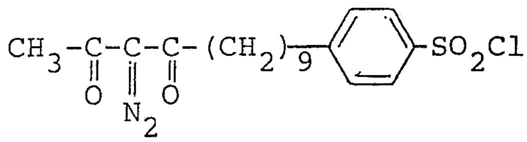 | " | " | " |
| 16 | 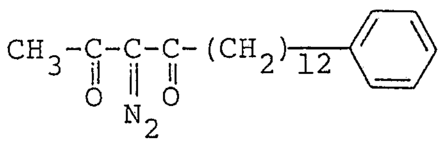 | 10 | 90 | 300 |
| 17 | 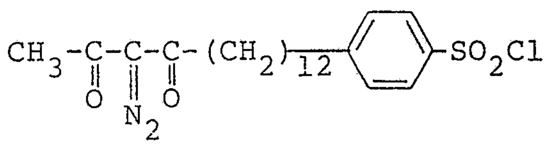 | " | " | " |
| 18 | 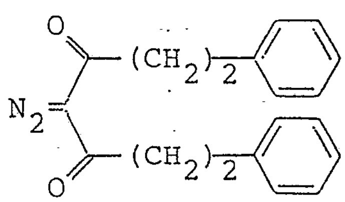 | 15 | 85 | 300 |
| 19 | 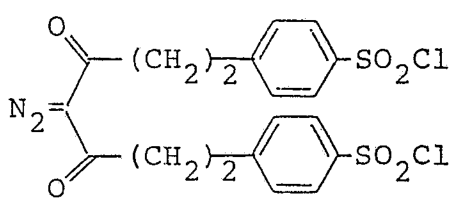 | " | " | " |

(To be cont'd)

Table 1 (Cont'd)

| | | | |
|---|---|---|---|
| 100 | 10 | 82 | 0.4 |
| 80 | 8 | 85 | 0.4 |
| 80 | 8 | 85 | 0.4 |
| 50 | 5 | 88 | 0.4 |
| 300 | 30 | 75 | 0.4 |
| ≦10 | 0 | 86 | 0.4 |

(To be cont'd)

Table 1 (Cont'd)

| | | | | |
|---|---|---|---|---|
| 20 | 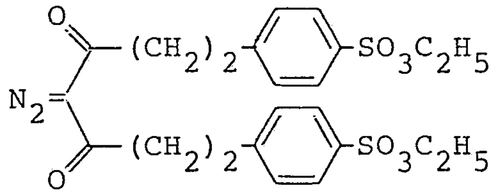 | 15 | 85 | 300 |
| 21 | 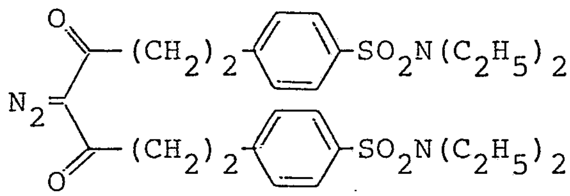 | " | " | " |
| 22 | 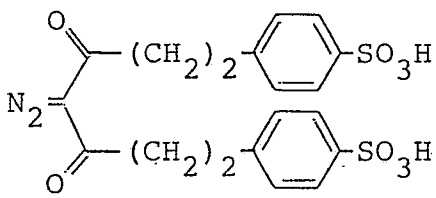 | " | " | " |
| 23 | 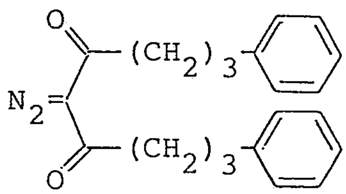 | 12 | 88 | 300 |
| 24 | 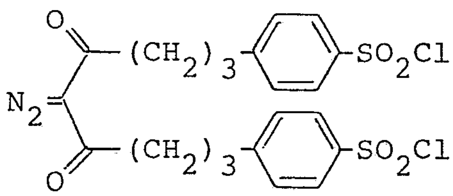 | " | " | " |

(To be cont'd)

Table 1 (Cont'd)

| | | | |
|---|---|---|---|
| 150 | 15 | 82 | 0.4 |
| 150 | 15 | 82 | 0.4 |
| 150 | 15 | 82 | 0.4 |
| 150 | 15 | 82 | 0.4 |
| ≦10 | 0 | 88 | 0.4 |

(To be cont'd)

Table 1 (Cont'd)

| | | | | |
|---|---|---|---|---|
| 25 | 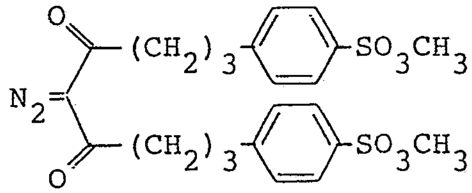 | 12 | 88 | 300 |
| 26 | 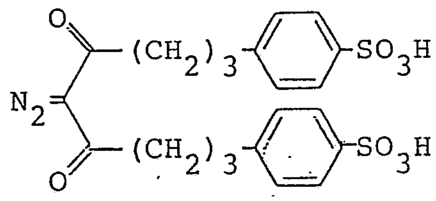 | " | " | " |
| 27 | 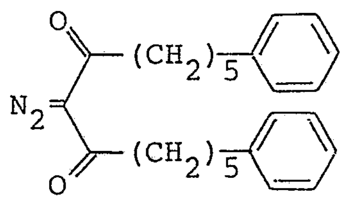 | 10 | 90 | 300 |
| 28 | 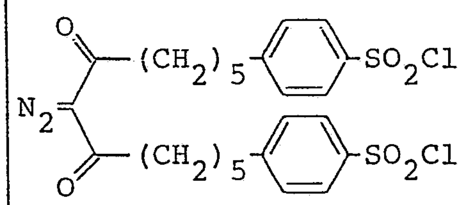 | " | " | " |
| 29 | 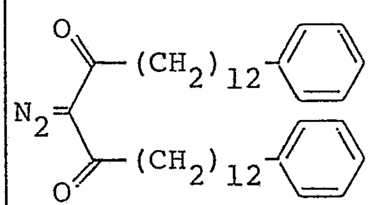 | " | " | " |
| 30 | 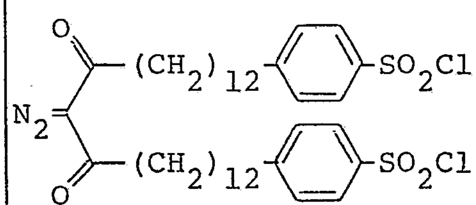 | 6 | 94 | 300 |

(To be cont'd)

Table 1 (Cont'd)

| | | | |
|---|---|---|---|
| 90 | 10 | 84 | 0.4 |
| 90 | 10 | 84 | 0.4 |
| 100 | 10 | 80 | 0.4 |
| ≦10 | 0 | 90 | 0.4 |
| ≦10 | 0 | 90 | 0.4 |
| ≦10 | 0 | 90 | 0.4 |

(To be cont'd)

Table 1 (Cont'd)

| | | | | |
|---|---|---|---|---|
| 31 | 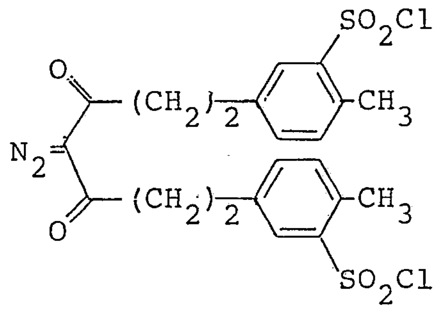 | 15 | 85 | 300 |
| 32 | 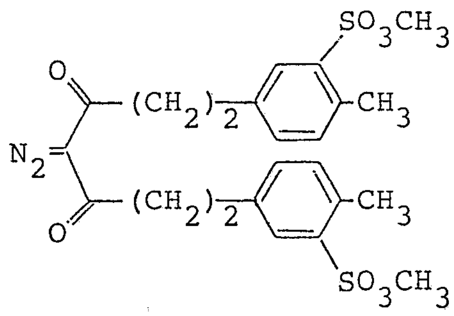 | " | " | " |
| 33 | 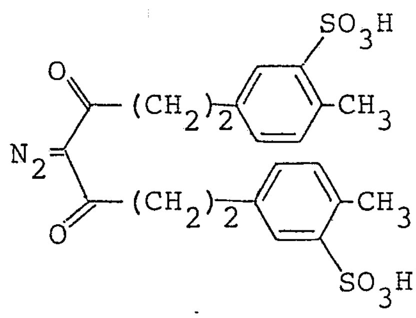 | " | " | " |
| 34 | 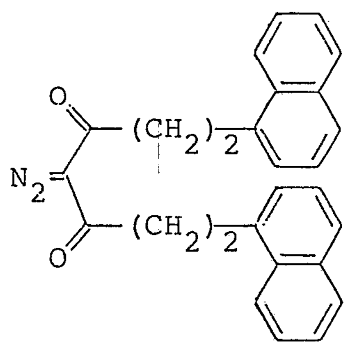 | 10 | 90 | 300 |

Table 1 (Cont'd)

| | | | |
|---|---|---|---|
| ≦10 | 0 | 90 | 0.4 |
| 100 | 10 | 85 | 0.4 |
| 100 | 10 | 85 | 0.4 |
| 80 | 10 | 83 | 0.4 |

(To be cont'd)

Table 1 (Cont'd)

| | | | | |
|---|---|---|---|---|
| 35 | 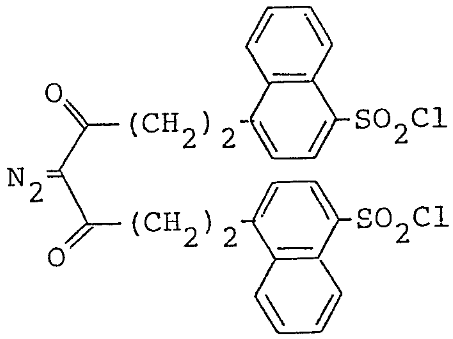 | 10 | 90 | 300 |
| 36 | 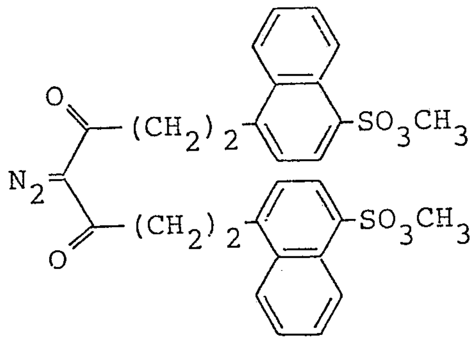 | " | " | " |
| 37 | 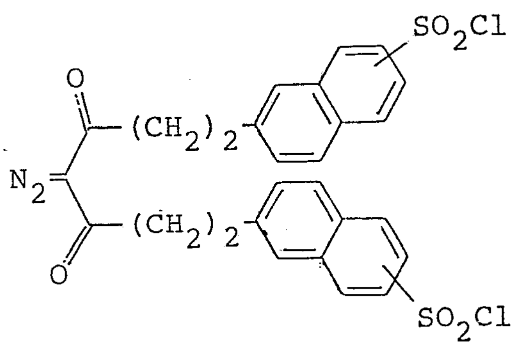 | " | " | " |
| 38 | 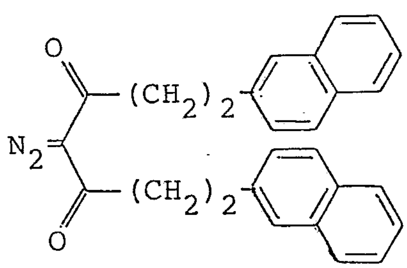 | " | " | " |

(To be cont'd)

Table 1 (Cont'd)

| | | | |
|---|---|---|---|
| ≦10 | 0 | 90 | 0.4 |
| 50 | 5 | 84 | 0.4 |
| ≦10 | 0 | 90 | 0.4 |
| 80 | 10 | 83 | 0.4 |

(To be cont'd)

Table 1 (Cont'd)

| | | | | |
|---|---|---|---|---|
| 39 | 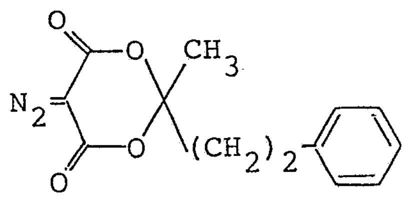 | 15 | 85 | 300 |
| 40 | 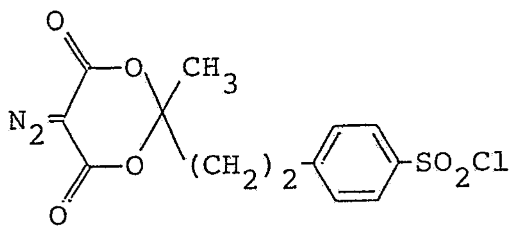 | " | " | " |
| 41 | 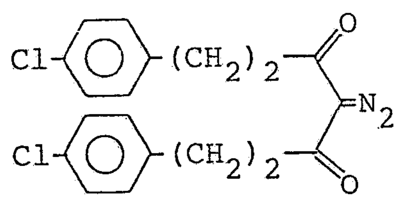 | " | " | " |
| 42 | 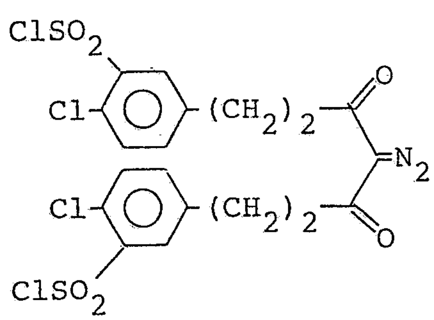 | " | " | " |

(To be cont'd)

Table 1 (Cont'd)

| | | | |
|---|---|---|---|
| 400 | 40 | 70 | 0.4 |
| 50 | 5 | 82 | 0.4 |
| ≦10 | 0 | 86 | 0.4 |
| <10 | 0 | 90 | 0.4 |

(To be cont'd)

Table 1 (Cont'd)

| | | | | |
|---|---|---|---|---|
| 43 | 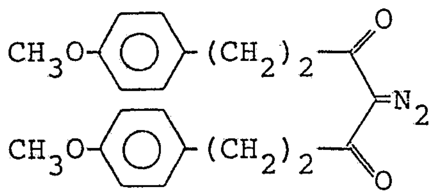 | 15 | 85 | 300 |
| 44 | 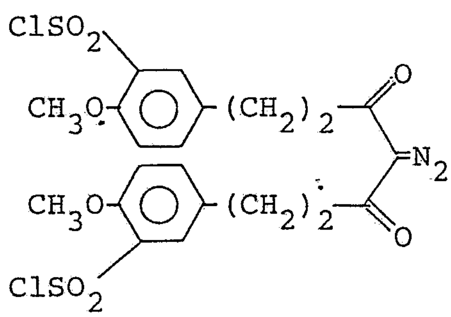 | " | " | " |

(To be cont'd)

Table 1 (Cont'd)

| | | | |
|---|---|---|---|
| 150 | 15 | 82 | 0.4 |
| <10 | 0 | 90 | 0.4 |

As is clear from Table 1, influences of the methylene chain length are shown in Run Nos. 2, 13, 15 and 17, Run Nos. 1, 12, 14 and 16, Run Nos. 18, 23, 27 and 29, and Run Nos. 19, 24, 28 and 30, wherein longer methylene chains provide good results with smaller using amounts, that is, the sensitivity as the photosensitive compound is increased.

Further, influences of substituents are shown in Run Nos. 1 and 2 to 11, wherein Run No. 2 is the best; in Run Nos. 18 and 19 to 22, wherein Run No. 19 is the best; and in Run Nos. 23 and 24 to 26, wherein Run No. 24 is the best.

## Claims

1. A photosensitive composition comprising a resin and a photosensitive compound of the formula:

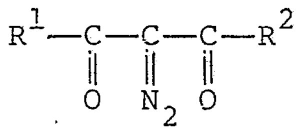 [I]

wherein $R^1$ is 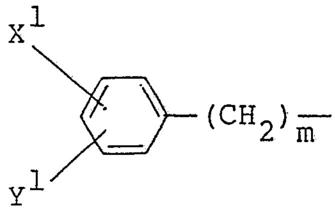 or 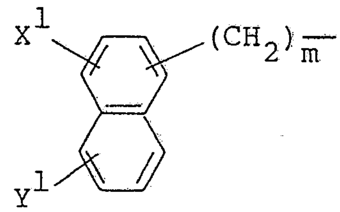 ;

$X^1$ and $Y^1$ are independently a hydrogen atom, a halogen atom, an alkyl group, an alkoxy group,

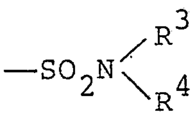 ,

$-SO_3R^5$ or a functional group reactive with the resin; $R^3$ and $R^4$ are independently a hydrogen atom or an alkyl group which may contain one or more functional groups, or $R^3$, $R^4$ and N taken together may form a heterocyclic ring; $R^5$ is an alkyl group; m is an integer of 1 or more; $R^2$ is an alkyl group, a cycloalkyl group, a hydroxyalkyl group, an alkoxyalkyl group,

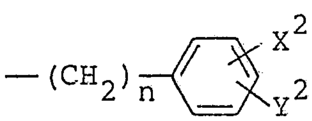 or 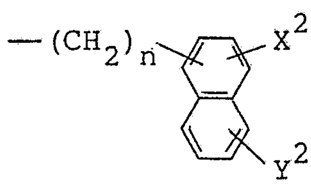 ;

$X^2$ and $Y^2$ are independently a hydrogen atom, a halogen atom, an alkyl group, an alkoxy group,

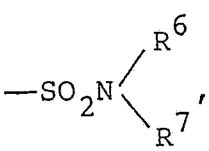 ,

$-SO_3R^8$ or a functional group reactive with the resin; $R^6$ and $R^7$ are independently a hydrogen atom or an alkyl group which may contain one or more functional groups, or $R^6$, $R^7$ and N taken together may form a heterocyclic ring; $R^8$ is an alkyl group; n is an integer of 1 or more; or $R^1$ and $R^2$ taken together may form a group of the formula:

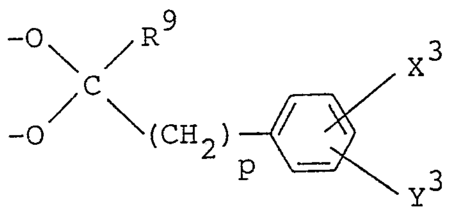

$R^9$ is an alkyl group, an aralkyl group, a hydroxyalkyl group, or an alkoxyalkyl group; $X^3$ and $Y^3$ are independently a hydrogen atom, a halogen atom, an alkyl group, an alkoxy group,

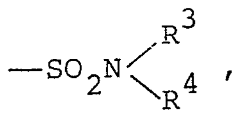

$-SO_3R^5$, or a functional group reactive with the resin; and p is an integer of 1 or more.

2. A photosensitive composition according to Claim 1, wherein the photosensitive compound is a compound represented by the formula:

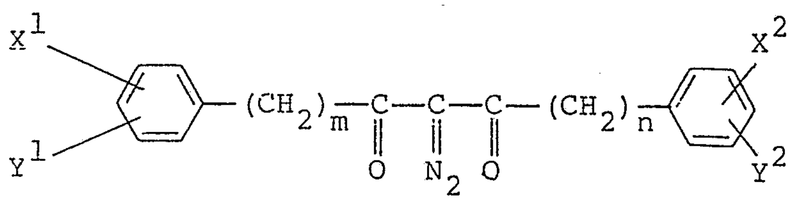

wherein $X^1$, $Y^1$, $X^2$, $Y^2$, m and n are as defined in Claim 1.

3. A photosensitive composition according to Claim 1, wherein the photosensitive compound is a compound represented by the formula:

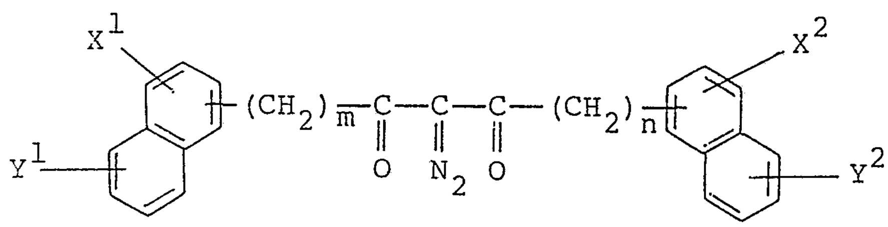

wherein $X^1$, $Y^1$, $X^2$, $Y^2$, m and n are as defined in Claim 1.

4. A photosensitive composition according to Claim 1, wherein the photosensitive compound is a compound represented by the formula:

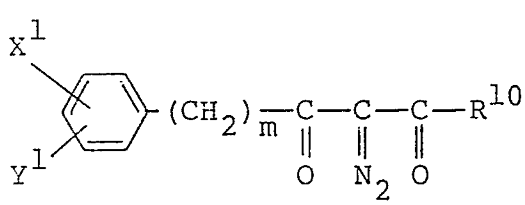

wherein $R^{10}$ is an alkyl group, a cycloalkyl group, a hydroxyalkyl group, or an alkoxyalkyl group; and $X^1$, $Y^1$ and m are as defined in Claim 1.

5. A photosensitive composition according to Claim 1, wherein the photosensitive compound is a compound represented by the formula:

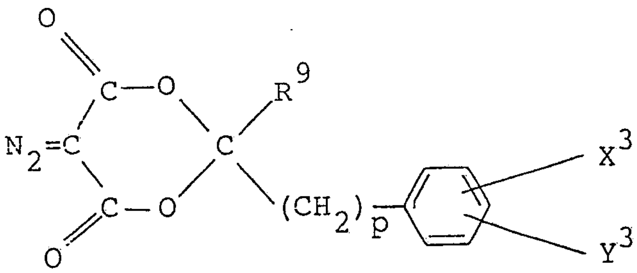

wherein $R^9$, $X^3$, $Y^3$ and p are as defined in Claim 1.

6. A process for forming a fine pattern which comprises
forming a film of a photosensitive composition comprising a resin and a photosensitive compound of the formula:

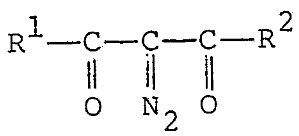

[I]

wherein $R^1$ and $R^2$ are as defined in Claim 1, on a substrate,
exposing the film to deep UV light of less than about 300 nm, and
subjecting to development to form a pattern by removing exposed portions of the film.

7. A process according to Claim 6, wherein the photosensitive compound is a compound represented by the formula:

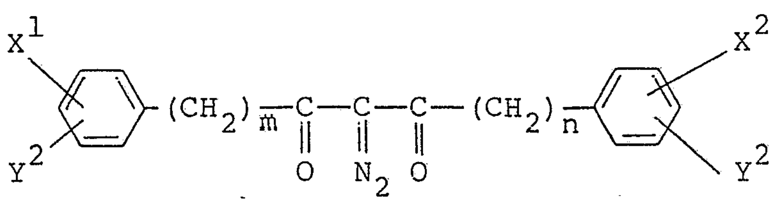

wherein $X^1$, $Y^1$, $X^2$, $Y^2$, m and n are as defined in Claim 6.

8. A process according to Claim 6, wherein the photosensitive compound is a compound represented by the formula:

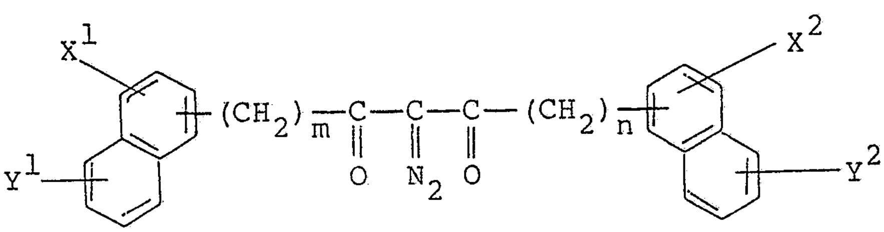

wherein $X^1$, $Y^1$, $X^2$, $Y^2$, m and n are as defined in Claim 6.

9. A process according to Claim 6, wherein the photosensitive compound is a compound represented by the formula:

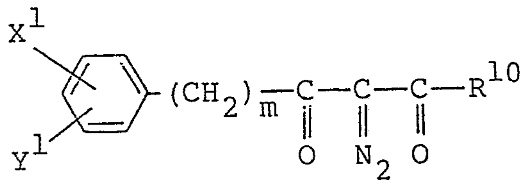

wherein $R^{10}$ is an alkyl group, a cycloalkyl group, a hydroxyalkyl group, or an alkoxyalkyl group; and $X^1$, $Y^1$ and m are as defined in Claim 6.

10. A process according to Claim 6, wherein the photosensitive compound is a compound represented by the formula:

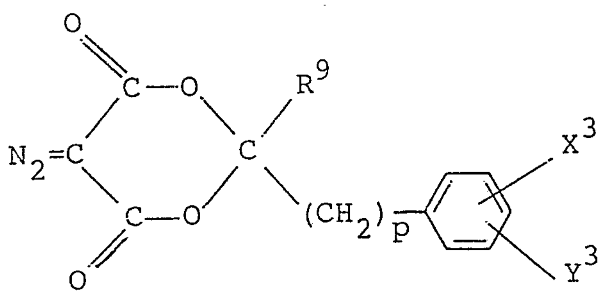

wherein $R^9$, $X^3$, $Y^3$ and p are as defined in Claim 6.

11. A process for forming a fine pattern which comprises
forming a photoresist layer on a substrate,
forming a contrast enhancing material layer comprising a resin and a photosensitive compound of the formula:

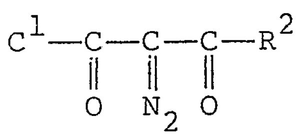

wherein $R^1$ and $R^2$ are as defined in Claim 1, on the photoresist layer,
exposing the contrast enhancing material layer to deep UV light of less than about 300 nm,
removing the contrast enhancing material layer, and at the same time,
developing the photoresist layer to form a fine pattern by removing exposed portions of the phtoresist layer.

12. A process according to Claim 11, wherein a water-soluble organic film is formed between the photoresist layer and the contrast enhancing material layer, and said water-soluble organic film is removed simultaneously with the contrast enhancing material layer.

13. A process according to Claim 11, wherein the photosensitive compound is a compound represented by the formula:

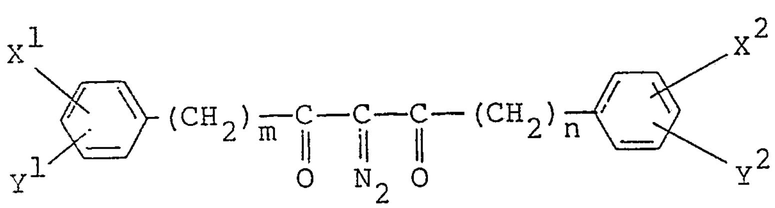

wherein $X^1$, $Y^1$, $X^2$, $Y^2$, m and n are as defined in Claim 11.

14. A process according to Claim 11, wherein the photosensitive compound is a compound represented by the formula:

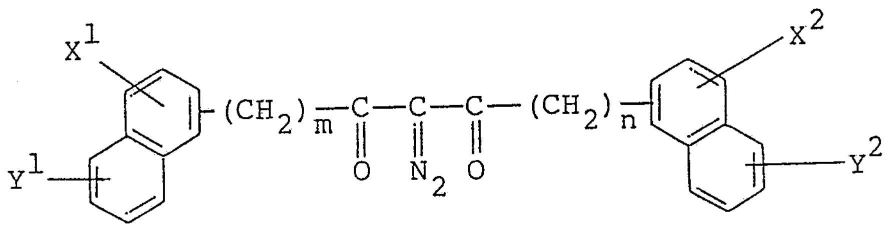

wherein $X^1$, $Y^1$, $X^2$, $Y^2$, m and n are as defined in Claim 11.

15. A process according to Claim 11, wherein the photosensitive compound is a compound represented by the formula:

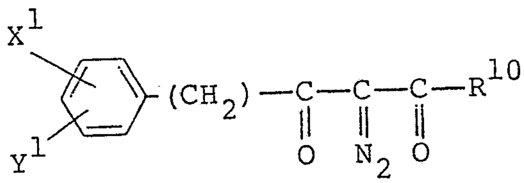

wherein $R^{10}$ is an alkyl group, a cycloalkyl group, a hydroxyalkyl group, or an alkoxyalkyl group; and $X^1$, $Y^1$ and m are as defined in Claim 11.

16. A process according to Claim 11, wherein the photosensitive compound is a compound represented by the formula:

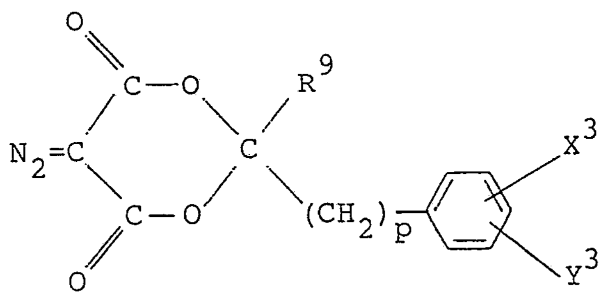

wherein $R^9$, $X^3$, $Y^3$ and p are as defined in Claim 11.

FIG.1(a)

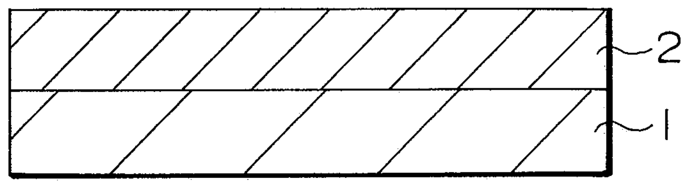


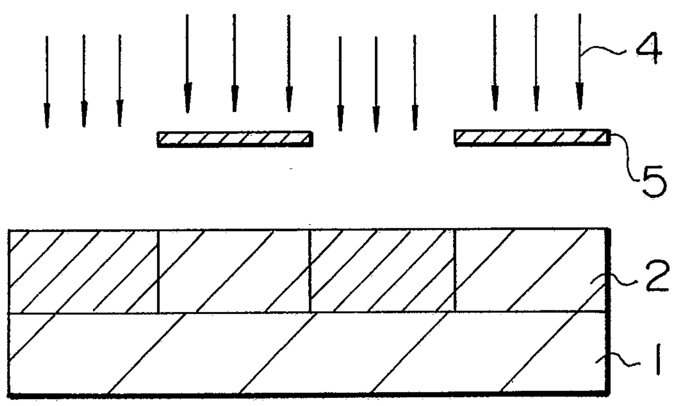


FIG.1(b)

FIG.1(c)

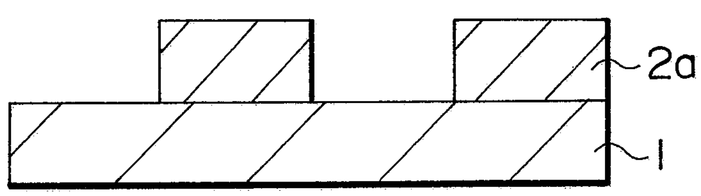

FIG. 2

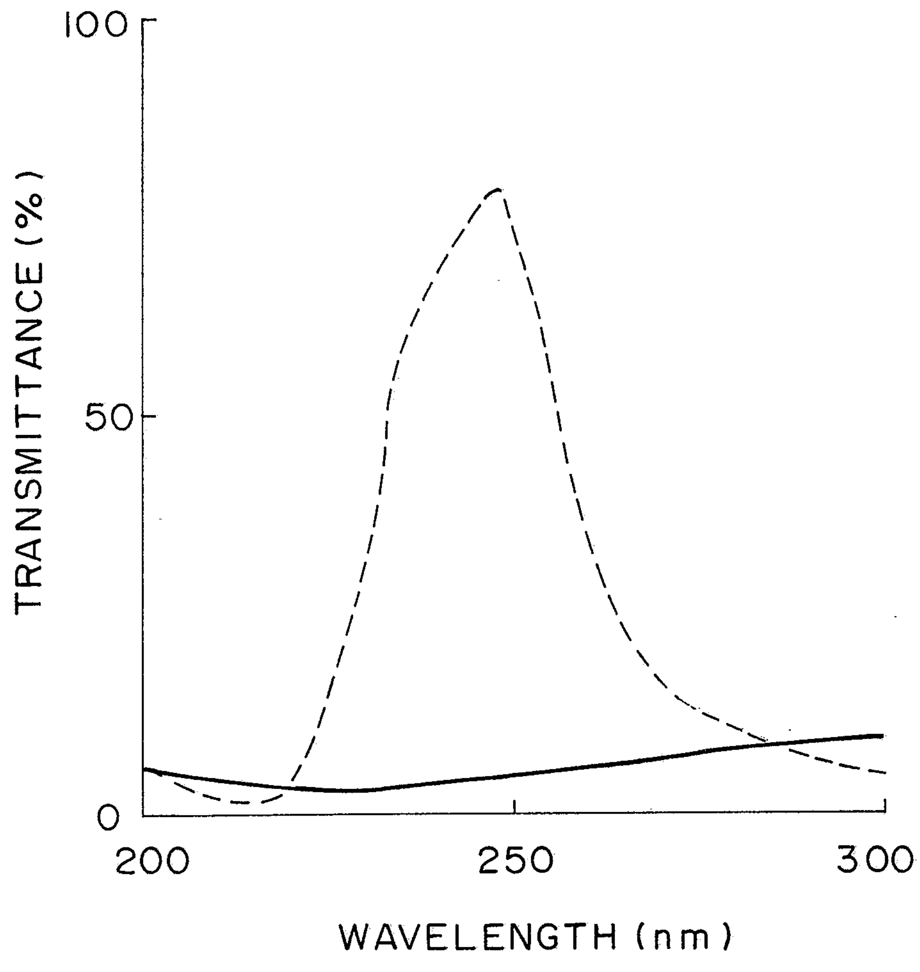

FIG. 3(a)

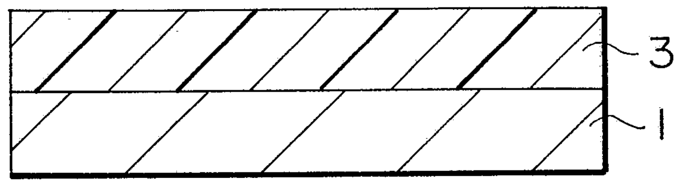


FIG. 3(b)

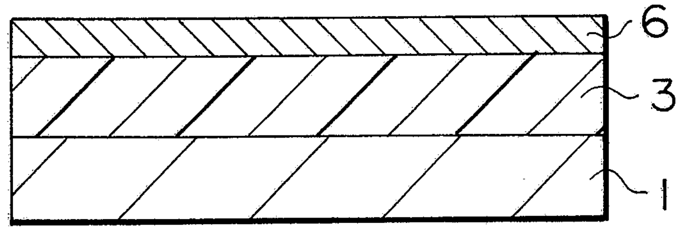


FIG. 3(c)

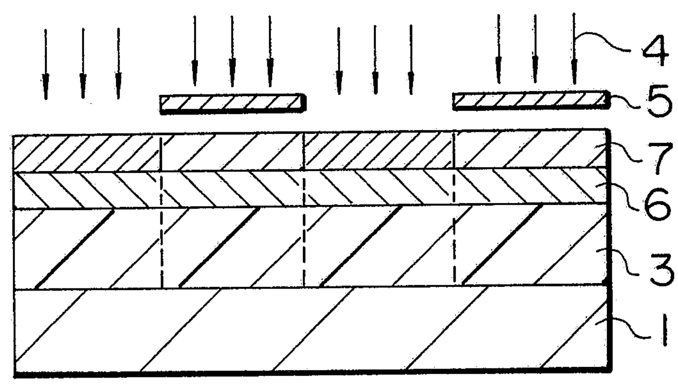


FIG. 3(d)

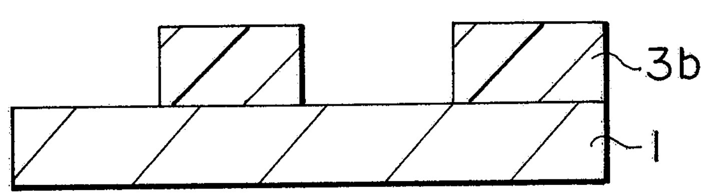

FIG. 4(a)

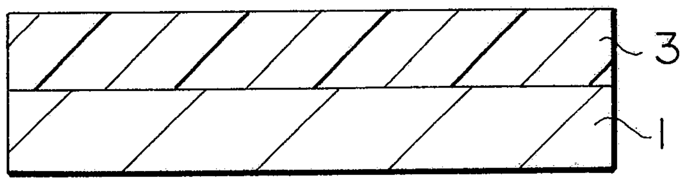


FIG. 4(b)

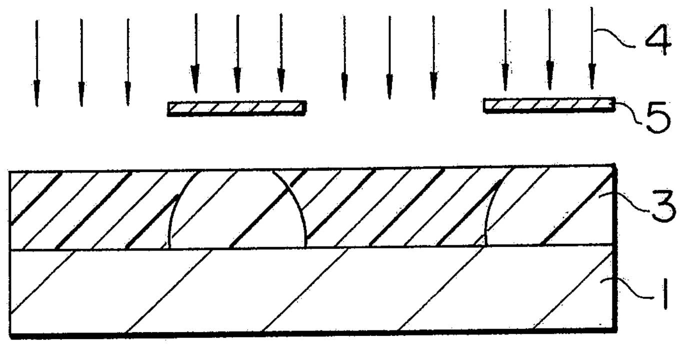


FIG. 4(c)

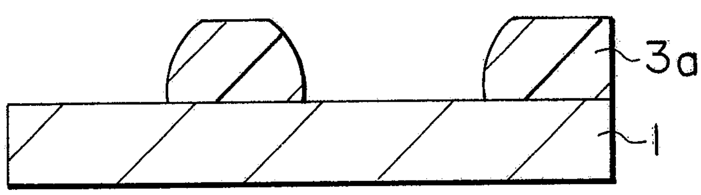

FIG. 5

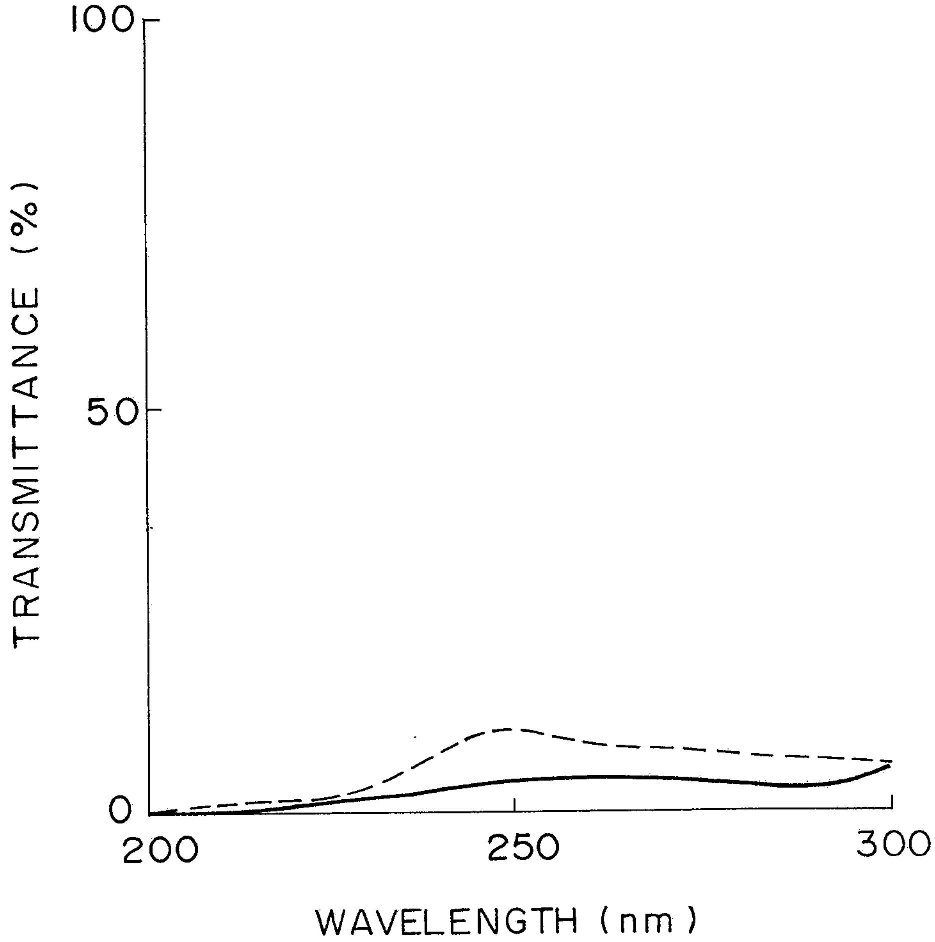

FIG. 6(a)

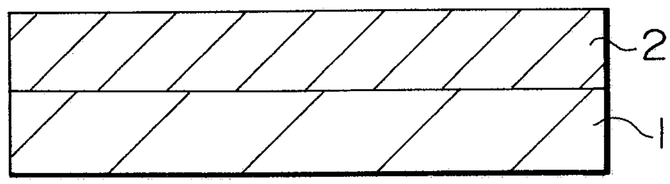


FIG. 6(b)

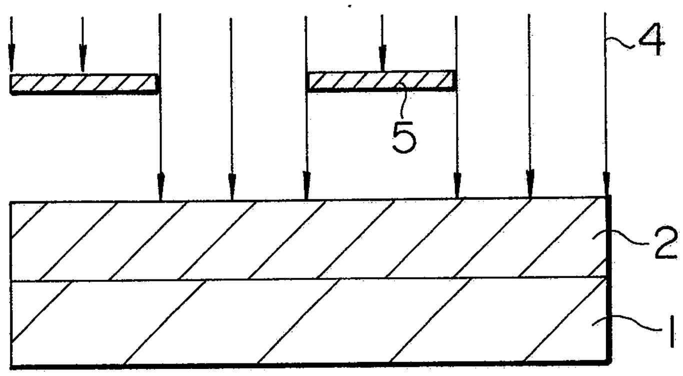


FIG. 6(c)

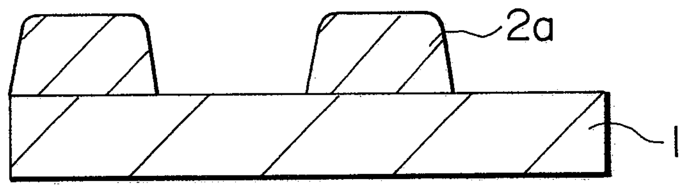

FIG. 7

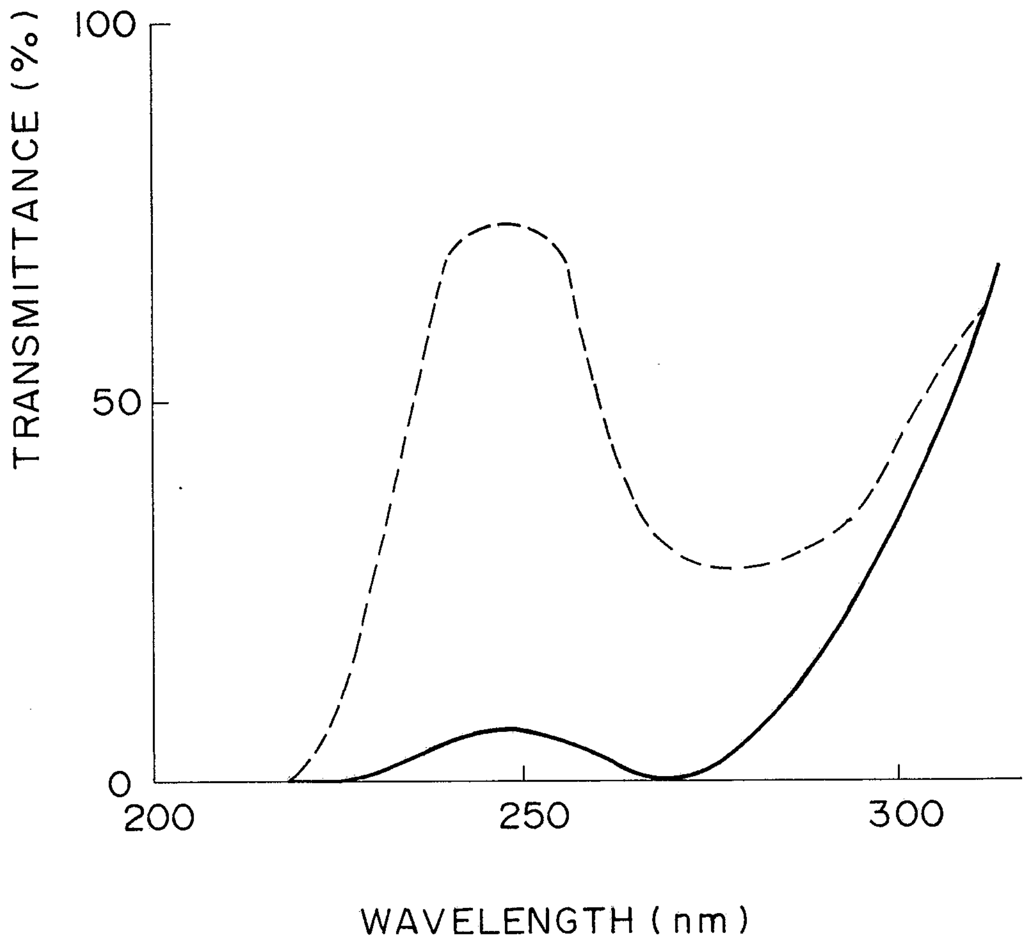

FIG. 8